# EUROPÄISCHE PATENTSCHRIFT

(11) **EP 4 222 299 B1**
(45) Veröffentlichungstag und Bekanntmachung des Hinweises auf die Patenterteilung: **06.11.2024**
(21) Anmeldenummer: 21794943.7
(22) Anmeldetag: 04.10.2021
(51) Int. Cl.: D04B 1/12, D04B 21/08, A61F 13/15, A61F 13/537

(54) **WIEDERVERWENBARE FEUCHTIGKEITSREGULIERENDE TEXTILE FLÄCHE**
REUSABLE MOISTURE-REGULATING TEXTILE SHEET
FEUILLE TEXTILE RÉUTILISABLE RÉGULANT L'HUMIDITÉ

(30) Priorität: 01.10.2020 DE 102020006022
(43) Veröffentlichungstag der Anmeldung: 09.08.2023
(73) Patentinhaber: MEDICAL SYSTEM PROTECT GMBH, 41066 Mönchengladbach (DE)
(72) Erfinder: BENDT, Ellen, 41066 Mönchengladbach (DE); RICHTER, Christoph, 41066 Mönchengladbach (DE); CHISI, Towela, 41066 Mönchengladbach (DE); BÜSCHKENS-GÖTZ, Ann-Kathrin, 41066 Mönchengladbach (DE)
(74) Vertreter: Dehns Germany Partnerschaft mbB
(86) Internationale Anmeldenummer: PCT/IB2021/022222
(87) Internationale Veröffentlichungsnummer: WO 2022/069950

(56) Entgegenhaltungen:
- EP-A1- 0 921 221
- EP-A1- 3 358 056
- WO-A1-2015/185652
- DE-A1- 10 055 902
- DE-A1- 102013 105 877
- DE-A1- 102014 108 009

## Beschreibung

Die vorliegende Erfindung betrifft eine textile Fläche, die ein gutes Flüssigkeitsmanagement aufweist.

Textile Flächen, also Flächengebilde aus Fasern oder Fäden werden zu Gestricken, Geweben, Maschenware, Geflechten oder auch zu Watte, Vliesstoffen und Filz verarbeitet Die Fasern oder Fäden werden gestrickt, gefilzt, gewebt, verkreuzt, verklebt und verhakt, um sie anschließend zu Stoffen zu verarbeiten, welche dann weiter zu Tüchern, beispielsweise Geschirrtüchern gewebt oder auch Kleidung weiter verarbeitet werden. Es können auch hochwertige Funktionstextilien entstehen, also Textilien aus Fasern, Garnen, Geweben, Gestricken und Gewirken mit einem funktionellen Mehrwert. Diese Funktionstextilien können die verschiedensten Eigenschaften aufweisen. Sie können unter anderem winddicht, wasserdicht, atmungsaktiv, thermoregulierend, schmutzabweisend, antimikrobiell, flammhemmend, UV-beständig, elektrisch abschirmend, elastisch, strapazierfähig, pflegeleicht, chemikalienresistent; leicht, wärmend/kühlend sein. Allein schon die hier aufgezählte hohe Anzahl der Eigenschaften zeigt die große Bedeutung und Nachfrage nach Textilien, welche eben für bestimmte Zwecke besondere Eigenschaften aufweisen sollten. So werden bei den Funktionstextilien auch Qualitäten miteinander kombiniert; welche man nicht erwartet. Die große Beliebtheit von atmungsaktiven, also durchlässigen und wasserdichten, also für Wasser undurchlässigen, Bekleidungen für Outdooraktivitäten belegt dies.

Die DE 10 2013 105 877 A1 offenbart einen ein- oder mehrlagigen Matratzen- oder Kissenbezugsstoff mit zumindest einer aus einem Polyestergarn bestehende Strick- oder Wirkwarelage beschrieben, wobei die Strick- oder Wirkwarelage aus einem vollständig antimonfreien Polyestergarn gestrickt oder gewirkt ist. Des Weiteren werden ein Matratzen- oder Kissenbezug sowie eine Matratzen- oder Kissenschutzauflage beschrieben.

Ein in der EP 0 921 221 A1 beschriebener Strickstoff umfasst eine Schicht aus hydrophilem Garn auf einer Seite des Stoffes und eine Schicht aus hydrophobem Garn auf der gegenüberliegenden Seite des Stoffes. Zwischen den hydrophilen und hydrophoben Garnschichten erstreckt sich eine säulengenähte Garnschicht mit geringer Dichte, die diese miteinander verbindet.

Ein Stoffverbund mit einer oberen und einer unteren Textilschicht, einer mikroporösen Beschichtung und einer Polyfaserzwischenschicht aus ist aus der DE 100 55 902 A1 bekannt.

Die DE 10 2014 108 009 A1 offenbart eine elastische Bandage, die (a) eine untere elastische Lage, (b) eine Kernschicht, die eine Vielzahl von mit den elastischen Lagen (a) und (c) verbundenen Abstandsfäden aufweist, und (c) eine obere elastische Lage, umfasst, wobei die Kernschicht (b) ferner eine Vielzahl von Kernschichtgarnen aufweist, die sich im Wesentlichen parallel zu den Ebenen der elastischen Lagen (a) und (c) erstrecken.

Schließlich wird gemäß der EP 3 358 056 A1 Es ein Rundstrickstoff mit mehrschichtiger Struktur bereitgestellt, der aus einem einzelnen Rundstrickstoff mit einer Schichtstruktur mit zwei oder mehr Schichten gebildet wird und sich durch bestimmte Parameter auszeichnet.

Die hier in dieser Schrift gelehrte textile Fläche soll grundsätzlich ein gutes Flüssigkeitsmanagement aufweisen. Flüssigkeitsmanagement bedeutet die Aufnahme einer Flüssigkeitsmenge und das Halten eben dieser Flüssigkeitsmenge durch das jeweilige Textil. Anwendungsbereiche können Bettlaken, OP- Tücher, Inkontinenzflächen und Hygiene- und Reinigungsartikel sein. Der Wasserverlust des Körpers im Schlaf, die Trockenlegung von nässenden Körperstellen, die Aufrechterhaltung der Mobilität bei Inkontinenz und der allgemeine Hygiene- und Reinigungsbedarf und deren offensichtliche Folgen verlangen nach Lösungen.

Zwar gibt es schon viele Produkte auf dem Markt, die hier Abhilfe versprechen, doch die meisten sind in vielen Punkten noch verbesserungsfähig.

So leiden in Deutschland schätzungsweise sechs bis acht Millionen Menschen an einer Harninkontinenz, die umgangssprachlich auch als "schwache Blase" bezeichnet wird. Weltweit sind ca. 200 Millionen Menschen betroffen. Eine Harninkontinenz liegt vor, wenn Urin unkontrolliert und unwillkürlich abgeht. Dabei fehlt oder mangelt es dem Körper an der Fähigkeit, den Blaseninhalt zu speichern. Betroffene können selbst nicht mehr steuern, wann Urin abgegeben wird. Von einer Harninkontinenz kann schon ein junger Mensch betroffen sein. Die Zahl der Betroffenen nimmt jedoch mit steigendem Alter zu.

Als Hilfsmittel für die Betroffenen werden sog. aufsaugende (Vorlagen und Windeln) oder ableitende (z.B. Katheder und Kondomurinale) Inkontinenzprodukte angeboten. Des Weiteren gibt es noch eine große Anzahl von Einwegprodukten.

Die Entwicklung einer Mehrweginkontinenzfläche wäre in diesem Fall ein zu beschreitender Lösungsweg. Die zu entwickelnde Fläche muss auch hier die schon oben angesprochenen unterschiedlichen bis gegensätzlichen Eigenschaften besitzen. 1m Hauptfokus steht natürlich ein gutes Flüssigkeitsmanagement. Aber auch der Trage- und Nutzungskomfort, die Waschbarkeit, gute olfaktorische Eigenschaften und auch die Nachhaltigkeit, nämlich der Verzicht auf Folien und Beschichtungen, die schwer zu entsorgen sind, sind wichtige zu beachtende Eigenschaften. Gleiche Anforderungen gelten auch für Bettlaken, die die Körperflüssigkeit, hier den Schweiß der Nutzer, aufnehmen sollen. Bei OP-Tüchern und Verbänden (z. Bsp. Wundverbänden) werden noch zusätzliche Anforderungen, welche Keimfreiheit und Ähnliches fordern, zu erfüllen sein. Doch auch hier ist das Flüssigkeitsmanagement von grundlegender Bedeutung. Generell ist die Ausscheidung von Körperflüssigkeiten eine vollkommen normale menschliche Eigenschaft. Allerdings kann dies in einigen Situationen, falls man nicht vorbereitet ist, unpassend und daher unangenehm sein. Unerwünschte Flecken und Gerüche durch Schweiß, Menstruation oder Inkontinenz können peinliche Gefühle hervorrufen. Im Markt vorliegende Produkte bestehen aus mehreren Schichten, wobei die Saugflächen der Mehrwegprodukte oftmals durch ein Zusammennähen verschiedener Materialien realisiert werden. Diese Schichten müssen aufwendig miteinander verbunden werden. Sie können bei Produktionsfehlern, beispielsweise schlechter Vernähung auch die gespeicherte Flüssigkeit wieder verlieren.

Es ist daher Aufgabe der vorliegenden Erfindung eine solide textile Fläche bereitzustellen, die Flüssigkeit absorbieren, transportieren und zurückhalten kann und zudem noch wiederverwendbar und waschbar ist. Des Weiteren wird ein Verfahren zur Herstellung dieser textilen Fläche und deren Verwendungsmöglichkeiten angegeben.

Diese Aufgabe wird durch eine textile Fläche gelöst, welche als dreidimensionales mehrschichtiges Strickkonstrukt vorliegt, die mindestens drei Schichten aufweist, die dadurch gekennzeichnet sind, dass die erste Schicht die Flüssigkeit aufnimmt und zur Zwischenschicht transportiert, wobei diese Schicht einen hydrophilen Abschnitt aufweist, die zweite Schicht, nämlich die Zwischenschicht, die Flüssigkeit aufnimmt und zurückhält, wobei die Zwischenschicht hydrophobe Querfäden aufweist, welche die erste und die dritte Schicht miteinander verbinden und ein hydrophiles Inlay ist und die dritte Schicht, die verhindert, dass die Flüssigkeit aus dem Stoff entweicht, wobei die Schicht einen hydrophoben Abschnitt aufweist.

Durch diese textile Fläche wird es gewährleistet, dass die verschiedenen Materialeigenschaften innerhalb der textilen Fläche vereint sind. Die erste Schicht nimmt die Flüssigkeit auf und transportiert sie zur zweiten Schicht, der Zwischenschicht. Diese erste Schicht besitzt einen hydrophilen Abschnitt. Diese erste Schicht ist diejenige, die im Falle einer Anwendung am menschlichen Körper, dem Körper zugewandt ist. Sie liegt direkt am Körper an. Sie sorgt für die schnelle Aufnahme der Flüssigkeit. Dieser hydrophile Abschnitt ist glatt gestrickt. Er weist folgende Eigenschaften auf, nämlich antibakteriell, geruchsneutral und hypoallergen. Er kann aus jeder Art von hydrophilem Garn oder hydrophiler Faser bestehen. Hier sind, nicht abschließend zu verstehen, folgende Stoffe aufgezählt: Tencel, Deocell, Deowool, Baumwolle, Modal, Bambus, Lyocell, Viskose und deren Kombinationen in beliebiger Zusammensetzung. Die erste Schicht hat somit Dochtwirkungs- und Trocknungseigenschaften. Bei bestimmten Anwendungen kann die erste Schicht auch hydrophobe Eigenschaften aufweisen.

Die zweite Schicht, auch als Zwischenschicht bezeichnet, da sie zwischen der ersten und dritten Schicht liegt, weist hydrophobe Querfäden auf. Sie selbst dient als hydrophiles Inlay, welche die Flüssigkeit aufnimmt und zurückhält. Die hydrophoben Querfäden allerdings verbinden die erste und dritte Schicht so miteinander, dass diese aneinanderhängen. Die Anordnung der Zwischenschicht zwischen der ersten und der dritten Schicht und die Verbindung der beiden Schichten über Querfäden kann unter Verwendung von Fanghenkeln vorgenommen werden. Die Zwischenschicht selbst besteht teilweise aus Inlayfasern und/oder Inlaygarnen. Sie werden ebenfalls durch die Querfäden fixiert und so an ihrem vorgesehenen Ort gehalten. In der Tat werden sie nur durch die Querfäden dort gehalten. Auch die Stoffe der Zwischenschicht mit hydrophilem Charakter haben antibakterielle, geruchsneutrale und hypoallergene Eigenschaften. Sie bestehen aus jeder Art von hydrophilem Garn oder hydrophiler Faser bestehen. Hier sind, nicht abschließend zu verstehen, folgende Stoffe mit hydrophilem Charakter aufgezählt: Tencel, Deocell, Deowool, Baumwolle, Modal, Bambus, Lyocell, Viskose, Natriumpolyarcylat, Kohlenstoffkristalle, Monofilamente, Multifilament, lose Fasern, Mikrofasern, **Holzfasern,** Garne aus Superabsorberfasern und deren Kombinationen in beliebiger Zusammensetzung. Der Querfaden, also der Verbindungsfaden, der in der Zwischenschicht verläuft, ist ebenfalls antibakteriell, geruchsneutral und hypoallergen. Er kann aus jeder Art Garn mit hydrophoben Eigenschaften bestehen, so beispielsweise Polyester, synthetischen Monofilamenten oder Multifilamenten (PET, PES, PL, PCDT, PA, PP), aber auch Naturstoffen wie Leinen oder Hanf.

Die dritte Schicht, die verhindert, dass die Flüssigkeit aus dem Stoff entweicht, weist einen hydrophoben Abschnitt auf. Sie ist die Schicht, welche im Falle einer Anwendung am menschlichen Körper vom Körper weg weist. Sie kommt mit dem Körper nicht in Berührung, im Gegensatz zu der ersten Schicht. Sie ist ebenfalls glatt gestrickt. Auch hier sollte das Material antibakterielle, geruchsneutrale und hypoallergene Eigenschaften haben. Es kann aus jeder Art von hydrophoben Garn oder hydrophiler Faser bestehen. Hier sind, nicht abschließend zu verstehen, folgende Stoffe aufgezählt: Wolle, Wollmischungen, Baumwolle, Polyamid, Polyester, Modal, Tencel, optional Schmelzgarn und deren Kombinationen in beliebiger Zusammensetzung.

Wichtig ist in diesem Zusammenhang, dass die erfinderische textile Fläche in ihrer Funktionsweise in Schichten aufgeteilt werden kann. Diese Schichten werden weiterhin noch in Abschnitte aufgegliedert, aber diese textile Fläche ist einlagig. Das vorliegende Konstrukt ist allerdings gestrickt und nicht mehr in seine Schichten aufteilbar. Es benötigt auch keine Kante, es wird also keine Kantenbearbeitung während des Strickens und auch nicht nach Beendigung des Strickprozesses vorgenommen. Es handelt sich somit um ein Material, welches als einlagiges Gestrick vorliegt und verwendet werden kann. Dies ist so in dieser Form noch nicht im Stand der Technik bekannt. Der Stand der Technik lehrt, wie schon oben erwähnt, Funktionsflächen, die in einen Stoff eingefügt werden, durch Einkleben, Einnähen oder sonst wie. Nimmt man die vorliegende erfinderische textile Fläche, so kann diese in jede mögliche Form konfektioniert werden ohne, dass sie nachträglich bearbeitet werden muss oder gar ihre Funktion einbüßt.

Die erfinderische textile Fläche wird gestrickt, somit handelt es sich bei dem Endprodukt um ein Gestrick oder ein gestricktes Konstrukt. Es handelt es sich also um eine Umschlingung von Fäden.

In einer weiteren Ausführungsform der erfindungsgemäßen textilen Fläche ist diese dadurch gekennzeichnet, dass die erste und die dritte Schicht jeweils mindestens zwei Abschnitte aufweist, wobei die erste Schicht einen oberen Abschnitt, der hydrophob ist, und einen unteren Abschnitt, der hydrophil ist und die dritte Schicht einen oberen Abschnitt, der hydrophob ist und einen unteren Abschnitt, der wasserdicht ist aufweist. Die beiden Abschnitte in den beiden Schichten (erste Schicht und dritte Schicht) sind jeweils glatt gestrickt und plattiert. Mit Hilfe der Plattiertechnik werden die jeweiligen Maschenschichten nochmals um mindestens eine weitere platzierte Materialschicht erweitert. Ein zweiter Faden wird in den jeweiligen Strickprozess miteingebracht.

Der obere Abschnitt (Plattierfaden) der ersten Schicht sollte wiederum antibakterielle, geruchsneutrale und hypoallergene Eigenschaften aufweisen. Er kann aus jeder Art von hydrophoben Garn (Faser) bestehen, so z. Bsp. synthetische Materialien wie PET oder PA oder Merinowolle, Wollmischungen oder andere hydrophobierte Materialien und deren Kombinationen in beliebiger Zusammensetzung.

Der untere Abschnitt (Plattierfaden) der dritten Schicht sollte ebenfalls antibakterielle, geruchsneutrale und hypoallergene Eigenschaften aufweisen. Die Forderung ist somit allgegenwärtig. Er kann aus jeder Art von hydrophoben Garn (Faser) bestehen, so z. Bsp. Wolle, Wollmischungen optional beides gefilzt oder weiterhin optional Schmelzgarn, Polyamid und deren Kombinationen in beliebiger Zusammensetzung. Sie können weiterhin laminiert und/oder harzbeschichtet (auslaufsicher) oder sonstwie beschichtet (auslaufsicher) sein.

Alle genannten Materialien und Materiallisten sind hier nicht abschließend zu verstehen, sie können durch andere Funktionsmaterialien ersetzt werden. Jede Art von Faser oder Garn kann jede Schicht und jedes Element des beschriebenen Konstrukts einnehmen, abhängig vom Anwendungsbereich, den gewünschten Eigenschaften für das Endprodukt und/oder denjenigen Eigenschaften, die sich für die Hersteller als wirtschaftlich vorteilhaft erwiesen haben. Das Konstrukt der vorliegenden Erfindung ist als Rahmen zu verstehen, in dem viele Variationen auftreten können, Parameter wie Dicke, Stichdichte, Materialprozentsätze, Gewicht usw. können in Abhängigkeit von der spezifischen Anwendung variieren. In ähnlicher Weise können Materialien entsprechend kombiniert werden, um die gewünschten Eigenschaften für die geeigneten Anwendungen zu erzeugen, sei es in Bezug auf Kleidungsstücke, Inkontinenz, Hygiene, Human- oder Tiermedizin oder auf andere Weise. Die vorliegende erfindungsgemäße textile Fläche kann allein oder in Verbindung mit anderen Stoffen verwendet werden oder auch als Membran wirken, die durch Nähen, Kleben oder auf andere Weise in andere Materialkonstrukte eingeführt und an diesen befestigt wird.

Die Dicke bzw. der Durchmesser der textilen Fläche kann 2 mm - 3 mm betragen. Sie ist damit sehr dünn. Bei besonderen Verwendungen kann beispielsweise die Menge an Füllmaterial die Dicke der textilen Fläche erhöhen. Versuche haben hier gezeigt, dass ein Fläche mit einer Dicke vom 5 cm zu fertigen ist. Die Haptik der textilen Fläche ähnelt eher einem textilen Stoff und nicht einer Windel. Dies ist bei einer Verwendung als Inkontinenzbekleidung gewünscht.

Hier noch einmal beschreibend das Grundkonzept, auf welchem dem Gegenstand der erfinderischen textilen Fläche basiert. Das Ziel der textilen Fläche mit gutem Flüssigkeitsmanagement, Waschbarkeit und damit auch verbunden Wiederverwendbarkeit sowie den geruchsneutralisierenden Eigenschaften führte zu den in den Ansprüchen offenbarten mehrschichtigen Bindungskonstruktionen. Hierzu stellten sich weitere Herausforderungen ein, wie ein Trage- und Nutzungskomfort, insbesondere bei Verwendung der textilen Fläche in Produkten mit Hautkontakt. Die körperseitigen Flächen sind hier unter anderem anschmiegsam und weich auszugestalten. Es wurde also eine textile Fläche gefordert, welche eine schnelle Aufnahme von Flüssigkeit oder einem flüssigen Medium gewährleistet und andererseits das Austreten der Flüssigkeit oder flüssigen Mediums auf der anderen Seite der textilen Fläche nicht zulässt. Dies kann leicht am Beispiel eines Schweißtuches gezeigt werden. Der Schweiß soll vom Körper weggeführt werden, aber nicht die Kleidung erreichen.

Die erfinderische textile Fläche ist ein waschbares und mehrschichtig gestricktes Textil. Es hat hochabsorbierende Eigenschaften. Die Kombination von Materialien mit entgegengesetzten Eigenschaften, nämlich Hydrophobizität und Hydrophilie führt in der gesamten Zusammensetzung zu porösen Schichten, die wässrige Flüssigkeiten vom Körper weg und in Richtung des saugfähigen Kerns des Gestrickes transferieren. Die Schichten und Abschnitte mit ihrem jeweiligen Material und die Physik des Flüssigkeitstransportes ist in der Beschreibung und den Beispielen ausführlich dargelegt worden. Die Produktzusammensetzungen können den jeweiligen Stoff-Anforderungen angepasst werden. Es können zusätzlich weitere Textilveredelungsverfahren zur einer Verbesserung der Eigenschaften der Fläche vorgenommen werden. So können antibakterielle und/oder geruchsneutrale bzw. geruchsschluckende Fasern eingestrickt werden. Dies kann für jede Schicht und jeden Abschnitt einzeln oder zusammen vorgenommen werden. So stellt das Einspinnen von Metallionenfäden zum Erhöhen des antibakteriellen Charakters der textilen Fläche kein Problem dar. Hochwertige Kohlenstoffkristalle und Kohlenstoffnetze sowie Naturstoffe wie die geruchsneutralen Yamwurzeln können Bestandteil der textilen Fläche sein und in jeder Schicht (Abschnitt) des gestrickten Konstruktes verwendet werden. Dieses Baukastensystem erlaubt die problemlose Erweiterung der Einsatz- und Funktionsmöglichkeiten. Eine Anpassung an verschiedene Produktgruppen ist leicht zu bewerkstelligen.

Die textile Fläche kann auf einer Flach- oder Rundstrickmaschine hergestellt werden. Das Strickverfahren verläuft kontinuierlich. Die dreidimensionale mehrschichtige Strickstruktur wird auf der Rundstrickmaschine oder auf der Flachstrickmaschine durch eine bestimmte Abfolge von Stichen/Schlaufen (Maschen) (also durch die Nadelbewegung werden Maschen) erzeugt. Eine erste Schicht (Masche) wird bei einer Flachstrickmaschine im vorderen Nadelbett, bei einer Rundstrickmaschine im Zylinder gestrickt. Hierbei wird sowohl ein hydrophobes als auch ein hydrophiles Garn verwendet. Das hydrophobe Garn ist nach außen gerichtet, also weist es zur Außenseite der textilen Fläche hin. Das hydrophile Garn zeigt nach innen, also zur Innenseite der textilen Fläche. Die beiden Garne werden gleichzeitig unter Verwendung eines Plattierungsträgers (also eines Fadenführers) gestrickt, um auf jeder Nadel einen plattierten glatten Stich (Masche) zu erhalten, bei dem die Nadeln eine vollständige Umdrehung ausführen, um eine komplette Schlaufe (Masche) zu erzeugen. Hiernach wird eine zweite Schicht gestrickt, die bei einer Flachstrickmaschine im hinteren Nadelbett, bei einer Rundstrickmaschine im Zifferblatt gestrickt wird. Hierbei wird ein hydrophobes Garn oder aber auch aufeinanderliegende hydrophobe Garne und niedrigschmelzender thermoplastischen Faden (z. Bsp. Schmelzgarn) verwendet. Im Falle des Vorhandenseins des niedrigschmelzenden thermoplastischen Fadens weist dieser vom Nadelbett bzw. dem Zifferblatt weg nach außen, während das hydrophobe Garn nach Innen weist. Im Strickprozess kann ein zweiter Faden mit eingebracht werden. Dieser legt den identischen Weg zurück. Die beiden Fäden werden Grundfaden und Plattierfaden genannt. Im Sinne dieser Anmeldung werden den Fäden diese Bedeutung kontextgebunden zugeordnet.

Entweder werden das einzelne hydrophobe Garn oder aufeinanderliegende hydrophobe Garne und niedrigschmelzender thermoplastischer Faden (z. Bsp. Schmelzgarn) unter Verwendung eines Plattierungsträgers (also eines Fadenführers) gestrickt, um auf jeder Nadel einen einfachen Stich (eine einfache Masche) zu haben, bei dem die Nadeln eine vollständige Umdrehung/Bewegung ausführen, um dann vollständige Schleifen/Schlaufen (Maschen) zu erzeugen. Hiernach wird/werden der/die Querfaden/Querfäden (Polfaden/Polfäden) in die textile Fläche eingestrickt, wobei die Querfäden (Polfäden) im Falle einer Flachstrickmaschine in den vorderen und hinteren Nadelbetten, im Falle einer Rundstrickmaschine im Zylinder und Zifferblatt gestrickt werden. Hierzu wird ein Standardträger (Standardstift oder Fadenführer) verwendet. Das hydrophobe Monofilament, welches die Querfäden bildet, wird zwischen den Nadeln angeordnet, wobei sich nur jede 4.te Nadel im vorderen und hinteren Nadelbett (im Falle der Flachstrickmaschine) oder im Zifferblatt und Zylinder (im Falle der Rundstrickmaschine) auf halber Strecke bewegt. Hierdurch wird der Henkel erzeugt, welcher die erste und dritte Schicht zusammenhält. Damit ein Zick-Zack-Bindungsbild erzeugt wird, sind die Nadeln, welche sich bewegen, im Nadelbett jeweils 2 Nadeln zueinander versetzt. Nun wird das hydrophobe Füllgarn zwischen die Schichten gebracht. Hierzu wird es zwischen den vorderen und hinteren Nadelbetten (im Falle der Flachstrickmaschine) oder dem Zylinder und dem Zifferblatt (im Falle der Rundstrickmaschine) mit Hilfe eines Einlegeträger (bsp. Inlay oder Fadenführer) angeordnet (platziert). Die Nadeln bewegen sich hierbei nicht.

In anderen Worten, um das Verfahren weiter zu verdeutlichen, verläuft der Strickprozess auf einer hierzu geeigneten Strickmaschine wie folgt: Die Nadel von dem vorderen Nadelbett oder die Nadel von dem hintern Nadelbett geht hoch, beim Fadenführen mit 2 Fäden werden beide Fäden in die Nadel gelegt. Dieser Prozessverlauf wird bei der 1. und 3. Schicht angewendet. Der Querfaden (Polfaden bspw. ein Monofilament) wird eingelegt, jede 4.te Nadel fährt halb hoch und halb runter. Das Füllmaterial wird eingelegt.

Dieser gesamte Vorgang wird solange wiederholt bis die textile Fläche fertig gestrickt ist. Das Füllmaterial kann nicht aus der textilen Fläche herausgenommen werden, da es von den **Polfäden** (Querfäden) gehalten wird. Anstelle jeder 4.ten Nadel, die den Querfaden führt, kann auch jede 3.te genommen werden. Hier aber liegen die Querfäden näher zusammen. Nimmt man jede 5.te Nadel, so könnte es Probleme beim Halten des Füllmaterials geben.

Natürlich wird die erfinderische textile Fläche auch über einen 3-D Textil-Drucker herstellbar sein.

Das Inlay, also die Füllschicht und das Monofilament, also die hydrophoben Querfäden bilden eigentlich eine Schicht aus, die aber aus 2 Materialien besteht. In dieser Anmeldung werden die 2 Materialien aber auch als 2 Schichten benannt, um die Wirkung zu verdeutlichen. Der Sinn erschließt sich aber aus dem Kontext.

Die Verwendung einer solchen textilen Fläche ist mannigfaltig. Sie kann überall eingesetzt werden, wo Flüssigkeiten oder flüssige Medien absorbiert und gespeichert werden sollen. Hierbei sind Hygiene, Geruchsneutralität, antibakterielle Eigenschaften, antiallergische Eigenschaften weitere Eigenschaften, die der textilen Fläche innewohnen oder innewohnen können. Der Aufbau der textilen Fläche läßt die Modifikation, die Verstärkung und die Anpassung einzelner Schichten an die jeweilige Verwendungsmöglichkeit zu. Der einfache Strickvorgang, welcher unmittelbar das jeweilige Produkt entstehen lässt und die fehlende Nachbearbeitung (wie das nachträgliche weitere Vernähen und Anbringen von Kanten) empfehlen eine Verwendung im medizinischen Bereich, im Pflegebereich und im dem Bereich der Hygiene und Haushaltshygiene. So sind Verwendungen in Kliniken als OP-Unterlagen, Bettunterlagen und Verbandsmittel denkbar. Des Gleichen gilt für den Veterinär-medizinischen Bereich. Hier sind insbesondere Tierdecken, OP-Unterlagen, Verbandsmaterial, Menstruationshosen zu nennen. Auch die Verwendung der textilen Fläche im täglichen Leben ist zu bedenken. Hier sind beispielsweise Gardinen, Tischdecken, Bezüge von Sitzmöbeln aller Art und Überwurfdecken zu nennen.

Die textile Fläche wird durch ein erfinderisches Verfahren hergestellt, welches dadurch gekennzeichnet, dass in einem ersten Schritt S1 eine erste Schicht aus einem hydrophoben und einem hydrophilen Garn gestrickt wird, wobei das hydrophobe Garn nach außen gerichtet ist und das hydrophile Garn nach innen weist und in einem zweiten Schritt S2 eine zweite Schicht aus einem hydrophoben Garn und/oder einem hydrophoben Garn zusammen mit einem niedrigschmelzenden thermoplastischen Faden gestrickt wird, wobei das hydrophobe Garn nach Innen weist und im Falle des Vorhandenseins der niedrigschmelzenden thermoplastischen Faden nach aussen weist, und folgend im Schritt S3 wird/werden der/die Querfaden/ Querfäden gestrickt; wobei sich nur jede 4.te Nadel auf halber Strecke bewegt, danach S4 wird das hydrophile Füllgarn zwischen den beiden Schichten platziert.

Eine weitere Ausführungsform des erfinderischen Verfahrens zur Herstellung einer wiederverwendbaren dreidimensionalen textilen Fläche (1) ist dadurch gekennzeichnet, dass nach den ersten beiden Schritten die Querfäden mit Versatz gestrickt werden. Es wird also der Befestigungspunkt der Polfäden gegeneinander verschoben. Der Versatz kann jede auch nur mögliche Verschiebung der Polfädenlagen gegeneinander annehmen. Auch die Abfolgeschritte S1, S2, S3 und S4 unterliegen nicht einem strengen Abfolgeplan, sondern können durchaus in unterschiedlicher Reihenfolge ablaufen. Dies gibt der textilen Fläche ebenfalls anderen Qualitäten und Eigenschaften. Die Polfäden, die in den nachfolgenden Figuren eine Winkel von 45 ° einschließen können ebenfalls in einem unterschiedlichen Winkelmaß gestrickt werden. Das unterschiedliche Winkelmaß wird durch ein Versetzen des Befestigungspunktes der Henkel zwischen den textilen Flächen erreicht Das resultierende Gewebe bleibt natürlich das Gleiche, aber Gleichmäßigkeit und Stabilität unter Druck sowie andere Eigenschaften verändern sich.

Stricken und Wirken sind zwei verschiedene Arten von Maschenwaren. Es handelt sich somit um Textilien, die durch Maschenbildung hergestellt werden.

Das gesamte Verfahrn kann durch einen Walkprozess abgeschlossen werden.

### Forschungsergebnisse (nicht erfindungsgemäß)

In Deutschland leiden schätzungsweise sechs bis acht Millionen Menschen an einer Harninkontinenz, die umgangssprachlich auch als "schwache Blase" bezeichnet wird. Weltweit sind ca. 200 Millionen Menschen betroffen? Eine Harninkontinenz liegt vor, wenn Urin unkontrolliert und unwillkürlich abgeht Dabei fehlt oder mangelt es dem Körper an der Fähigkeit, den Blaseninhalt zu speichern, und Betroffene können selbst nicht mehr steuern, wann Urin abgegeben wird. Bereits junge Menschen können von einer Harninkontinenz betroffen sein. Die Zahl der Betroffenen nimmt mit steigendem Alter jedoch zu.

Es gibt eine ganze Reihe unterschiedlicher Ursachen, die zu einer Harninkontinenz führen können. Diese können neurologische und/oder organische Ursachen haben. Dazu gehören Operationen am Unterleib, Schwangerschaften, bestimmte Grunderkrankungen wie Diabetes, aber auch Nervenschädigungen. Je nachdem, welche Ursachen zugrunde liegen, werden unterschiedliche Harninkontinenzformen unterschieden: Dranginkontinenz, Stressinkontinenz, Extraurethrale Inkontinenz, Reflexinkontinenz, Giggle Inkontinenz, Überlaufinkontinenz und Mischinkontinenz.

Zudem werden unterschiedliche Schweregrade von Inkontinenz, abhängig von der ungewollt austretenden Urinmenge, unterschieden:
Schweregrade von Inkontinenz
Leichte Inkontinenz: Ein paar Tropfen Urin zwischen den Toilettengängen bis hin zu 100 ml in 4 Stunden.
Empfohlene Produktionssaugfähigkeit: 150 ml bis 300 ml.
Mittlere Inkontinenz: unregelmäßiger Abang von Urinmengen bis 200 ml in 4 Stunden. Empfohlene Produktionssaugfähigkeit: 300 ml bis 700 ml.
Schwere Inkontinenz: Sehr große Blasenentleerung von mehr als 200 ml in 4 Stunden. Empfohlene Produktionssaugfähigkeit: über 1000 ml.
Sehr schwere Inkontinenz: Der gesamte Blaseninhalt entleert sich unkontrolliert..
Empfohlene Produktionssaugfähigkeit: über 1500 ml.

Als Hilfsmittel für die Betroffenen werden sog, aufsaugende (Vorlagen und Windeln) oder ableitende (z.B. Katheder und Kondomurinale) Inkontinenzprodukte angeboten.

Dieser vorliegende Bericht fokussiert sich auf die Entwicklung einer Mehrweg-Inkontinenzfläche. Diese Flächen stellt sich der Schwierigkeit, eine mehrschichtige Flächenkonstruktion zu entwickeln mit unterschiedlichen bis gegensätzlichen Eigenschaften in den einzelnen Materialschichten. Im Hauptfokus steht ein gutes Flüssigkeitsmanagement (Flüssigkeit aufnehmen und halten), in Kombination mit Trage-/Nutzungskomfoit, Waschbarkeit, guten olfaktorischen Eigenschaften und allen voran ein nachhaltiges Produkt (Verzicht von Folien und Beschichtungen).

### 2. Testung von Einweg- und Mehrwegprodukte

Das Testen der Einweg- und Mehrwegprodukte wird in Anlehnung an eine standardisierte MDS-Prüfmethode durchgeführt. Prüfmethode Nr. 12/2015 MDS-Hi "Prüfung von saugenden Inkontinenzhilfen".

Alle Prüfungsparameter wurden an die vorhandenen Monitorgrößen angepasst.

### 2.1 Test: Bestimmung der Flüssigkeitsaufnahme

Bei der Bestimmung der Flüssigkeitsaufnahme wird ein Urinersatz als Flüssigkeit genommen. Dieser soll die Viskosität von Urin nachbilden, um realitätsnah testen zu können. Die Flüssigkeitsaufnahme ist bei Inkontinenzprodukten ein wichtiges Indiz für die Produkt-Performance.

Das Prüfmaterial wird in 6 Proben á 4×8 cm geschnitten und beschriftet.
a) Der Urinersatz wird als Lösung hergestellt aus 3,6 g Salz und 400 ml Wasser.
b) Trockenes Probengewicht ermitteln.
c) Probe in Urinersatz tauchen und mit einer 280g schweren Druckplatte 5 Minuten beschweren.
d) Die Probe aus dem Urinersatz entnehmen und auf einen Sieb legen. Unter Einwirkung von 3,2 kg auf der Druckplatte für eine Minute beschweren. Anschließend die Probe wiegen.

### 2.2 Test: Bestimmung der Flüssigkeitsabgabe

Ein weiterer Test zur Bestimmung der Produkt-Performance ist der Test für die Flüssigkeitsabgabe. Inkontinenzhilfen sollen im Alltag unauffällig und bequem eingesetzt werden, weshalb auch das Testverfahren alltagsnah dargestellt werden muss. Bevor dieser Test durchgeführt werden kann, müssen die zuvor genutzten Proben wieder ganz trocken sein, deshalb gibt es eine Trocknungszeit von 48 Stunden (Angleichung der Raumfeuchte und Temperatur).

### Methode:

a) Mische 400ml Wasser mit 3,6g Salz zur Herstellung vom Urinersatz. Probe in Prüfgestell legen und mit 15ml Urinersatz beaufschlagen.
b) Die Probe mit Druckpolster und Druckplatte (1 ,28 kg) für 5 Minuten beschweren.
c) 4x Filterpapier mit einer Probengröße von 4x8cm abwiegen.
d) Der Versuchsaufbau wird folgendermaßen geschichtet: Probe -> 4x Filterpapier -> Druckpolster -> Gewicht.
e) Das Gewicht muss 2 Minuten auf dem Versuchsaufbau veniveilen. Anschließend die Filterpapiere erneut wiegen.

### 3. Marktübersicht Einwegprodukte

Vorlagen für eine leichte Inkontinenz bestehen üblichenweise aus Einwegmaterialien, die nur für den einmaligen Gebrauch konzipiert sind. Der typische Aufbau sieht wie folgt aus:

### Körperseitig beginnend:

Vliesabdeckung
Verklebung
Verteilerauflage
Saugkern: Cellulose plus Superabsorbergianulat
Verklebung
Wäscheschutzfolie (meist atmungsaktiv)
Haftkleber
Silikonpapier, abziehbar liegt dann an der Körperabgewandten Seite

Die Qualität eines Inkontinenzproduktes hängt dabei typischerweise neben dem Tragekomfort und der diskreten Optik, von der Qualität des sog. Saugkerns ab, der aus einem Gemisch von Zellstoff und sogenanntem Superabsorber besteht. "Er macht ungefähr 70 Prozent des Gesamtgewichts eines Inkontinenzproduktes aus und hat eine unterschiedlich hohe Speicherkapazität. Die Zellstofffasern verteilen die Flüssigkeit, die der Superabsorber dann aufnimmt. Der Superabsorber besteht aus teilneutralisiertem Natrium-Polyacrylat (mit hautneutralem pH-Wert). Sein Speichervermögen bei entsalztem Wasser ist das bis zu 500-fache des eigenen Gewichts und bei Urin immer noch die 30- bis 40-fache Menge. In trockenem Zustand handelt es sich um ein Granulat. Nach Aufnahme von Flüssigkeit wird es in ein Gel umgewandelt, indem es die Flüssigkeit im inneren seiner Struktur bindet. Dies sorgt für ein trockenes und hautfreundliches Gefühl. Unangenehme Gerüche werden vermieden und durch den leicht sauren pH-Wert des Granulats weiter reduziert; dies beruht vor allem auf der Hemmung des Bakterienwachstums. Ein Hauptbestandteil des Stoffwechsels der verbleibenden Bakterien ist stark riechender Ammoniak, der ebenfalls durch den niedrigen pH-Wert abgepuffert wird. Dies gilt eingeschränkt auch für Amine. Einige Produkte enthalten zudem Additive zur Geruchsverminderung. Ein weiterer wichtiger Qualitätsfaktor für Inkontinenzprodukte ist neben dem geringen Volumen die hohe Widerstandsfähigkeit gegen Druck. Auch dies ist ein Vorteil von Polyacrylat gegenüber Zellulose. Selbst unter starkem Druck, u.a. wenn das Körpergewicht im Sitzen oder Liegen auf dem Inkontinenzprodukt lastet, hält das Material die Flüssigkeit fest.

In ersten Schritt wurden die im Handel üblichen Einwegprodukte hinsichtlich ihrer Eigenschaften getestet, um einen Überblick über die Eigenschaften der verschiedenen Produkte zu bekommen. Diese werden dann später mit den im Handel erhältlichen Mehnivegprodukten verglichen. Wichtig bei dem Vergleich der Eigenschaften ist die Flüssigkeitsaufnahme und Flüssigkeitsabgabe der verschiedenen Einweg- und Mehrwegprodukte.

### 5. Neuentwicklung einer saugfähigen Fläche

### 3.1 Grundkonzept für den Flächenaufbau:

Für die Konzeption einer Fläche mit dem gewünschten Anforderungsprofil, wurden die bisher im Markt üblichen Mehrwegprodukte begutachtet und es wurde festgestellt, dass die eingesetzten Saugflächen meist durch ein Zusammennähen von verschiedenen Materialien realisiert wird. Dieser konfektionierte Schichtaufbau soll mit einer mehrschichtigen Bindungskonstruktion in Kombination mit neuen Materialien, ersetzt werden. Die Herausforderung ist es, gegensätzliche Materialeigenschaften innerhalb einer textilen Fläche zu vereinen: Eine schnelle Aufnahme von Flüssigkeit auf der Körperseite einerseits und ein Austreten der Flüssigkeit auf der anderen Bindungsseite (zur Bekleidung hin) andererseits. Angestrebt wird dies durch mehrere, unterschiedliche Materialschichten innerhalb eines Abstandsgestrickes. In der unten gezeigten Abbildung werden schematisch die verschiedenen Schichten der geplanten Fläche im Querschnitt dargestellt.

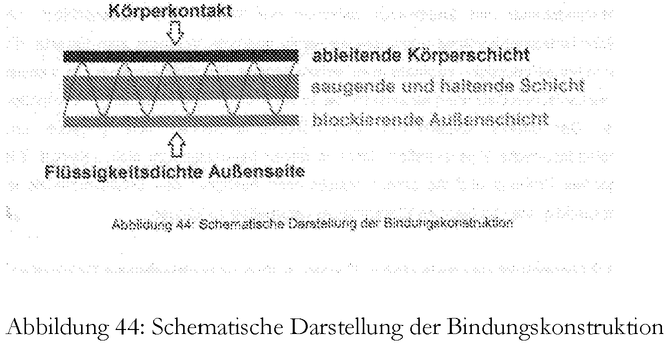

Grundsätzlich geht es um 3 Schichten mit unterschiedlichen Funktionen: Für die Außenschicht (vom Körper weg) wird eine filzfähige Wolle gewählt, die sich in den nachfolgenden Ausrüstungsprozessen verdichtet (filzt). Dieses Filzen sorgt im Zusammenhang mit den natürlichen hydrophoben Eigenschaften der Wolle, für eine wasserdichte Fläche und ist somit für die flüssigkeitsdichte Außenseite geeignet. Die Seite, welche den Körperkontakt hat, soll die anfallende Flüssigkeit schnellstmöglich ins Innere des Mehrlagengestrickes transportieren. Um die flüssigkeitstransportierende Körperschicht möglichst dünn zu gestalten, wird die Plattiertechnik angewandt, anhand derer zwei Garne innerhalb der Schicht nochmals getrennt positioniert werden können: Das flüssigkeitstransportierende Garn wird dabei außen und das erste flüssigkeitshaltende Garn innen venivendet wird. Das flüssigkeitstransportierende Garn außen dient zudem dazu, ein möglichst trockenes Gefühl auf der Haut zu erzeugen und den Flüssigkeitsrücktransport zu verhindern. Die Mittelschicht ist ebenfalls aus zwei Garnen konzipiert: Das sog. Verbindungsgarn, hier ein PES-Monofilament, hat zwei Funktionen in dem hier zu entwickelnden Mehrlagengestrick:
1. Es verbindet die leitende Körperschicht mit der flüssigkeitsdichten Außenschicht.
2. Es soll durch eine dosierte Steifigkeit für die Dimensionsstabilität der Fläche sorgen, um eine ungewollte Flüssigkeitsabgabe zu minimieren (z. B. durch Krafteinwirkung beim Sitzen).

Zu beachten ist dabei: Eine zu hohe Steifigkeit sorgt für eine nicht akzeptable Haptik, welche bei Inkontinenzprodukten von hoher Bedeutung ist. Das zweite Material ist das sog. Schussmaterial: Das Schussmaterial befindet sich als Abstandshalter und Saugkörper zwischen der Körper- und Außenschicht. Als Schussmaterial kommen vorzugsweise wenig gedrehte Vorgarne zum Einsatz, die eine hohe Feuchtigkeitsaufnahme aufweisen können. Das Schussmaterial wird keiner mechanischen Belastung ausgesetzt, da es von Körper- und Außenschicht umgeben ist. Der direkte Körperkontakt des Schussmaterials ist nicht gegeben und hautsensorische Eigenschaften daher in dieser Materialschicht nicht relevant. Die geringe Drehung und die daraus resultierende Festigkeit des Schussmaterials ist notwendig, um das Garn im Strickprozess verarbeiten zu können.

### 3.2 Übersicht der getesteten Garne in den verschiedenen Schichten:

Grundsätzlich sind bei der Garnauswahl die Anforderungsprofile eines Inkontinenzmehnivegproduktes als Ganzes und der einzelnen oben beschriebenen Materialschichten im Detail zu beachten. Hydrophile und hydrophobe Materialien für das Feuchtigkeitsmanagement werden ebenso benötigt, wie weiche und feste Materialien für ein ausgewogenes Verhältnis zwischen Schmiegsamkeit und Stabilität. Weitere Herausforderungen liegen bei der möglichst hohen Möglichkeit der Geruchsneutralisation, da es beim Tragen des Produktes nicht zu unangenehmen Geruchsentwicklungen kommen soll. Neben der von Natur aus geruchsneutralisierenden Wolle, ist hier besonders die Regeneratfaser Lyocell interessant, deren antibakterielle Eigenschaften sich olfaktorisch günstig auswirken.

Die Möglichkeit ein Garn aus Fasern mit Superabsorber-Eigenschaften herzustellen wurde ebenfalls getestet. Die Fasereigenschaften gestalten den Spinnprozess sehr schwierig, die Fasern haben sich nur bedingt und nur anteilig in Mischungen mit anderen Materialien überhaupt verarbeiten lassen, im Rahmen dieses Projektes waren daher nur Erstversuche möglich.

### 5.1 Grundkonzept für den Flächenaufbau

Folgende Garnverteilung liegt der folgenden Entwicklung zugrunde. Zu beachten ist, dass es mehrere Materialschichten in den 3 Funktionsschichten gibt:

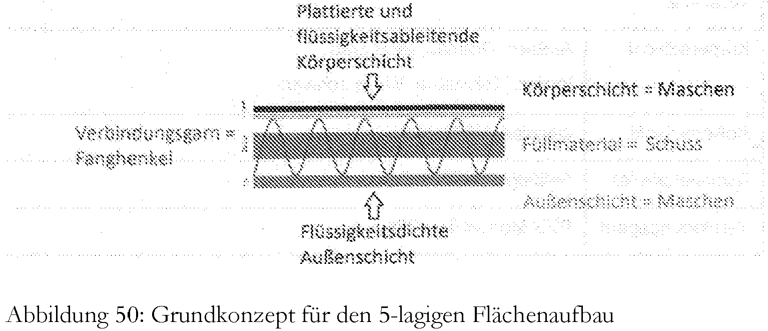

Optional kann die Außenschicht auch plattiert sein, sodass insgesamt 6 Schichten entstehen.

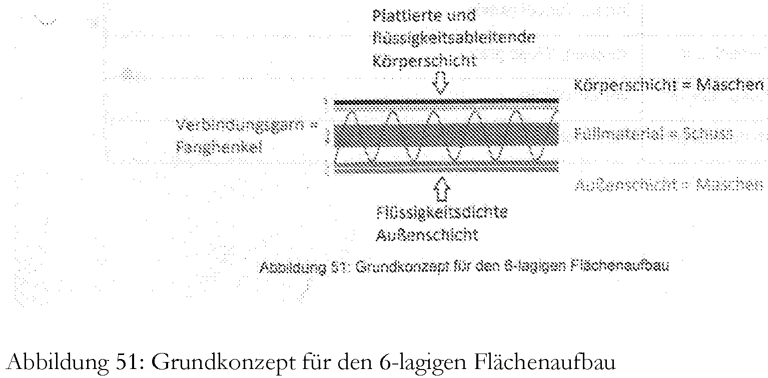

Folgende Garn wurden in den einzelnen Schichten gelestet:

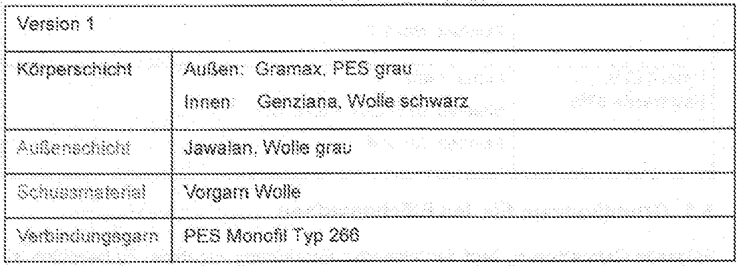

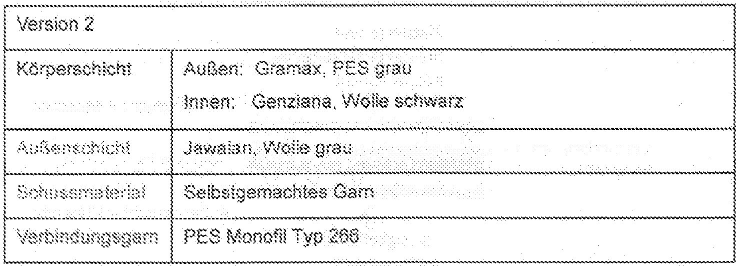

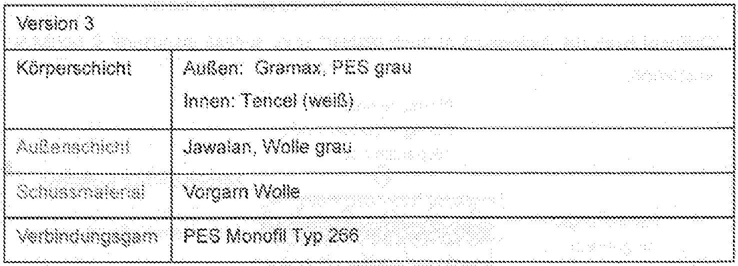

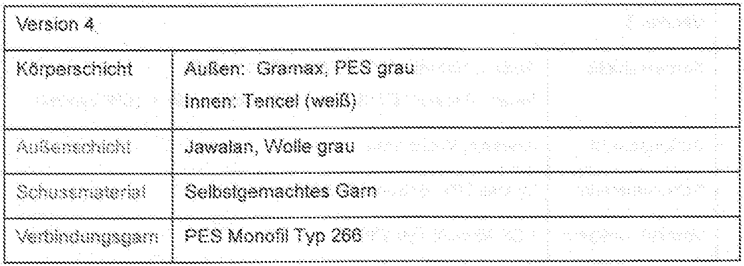

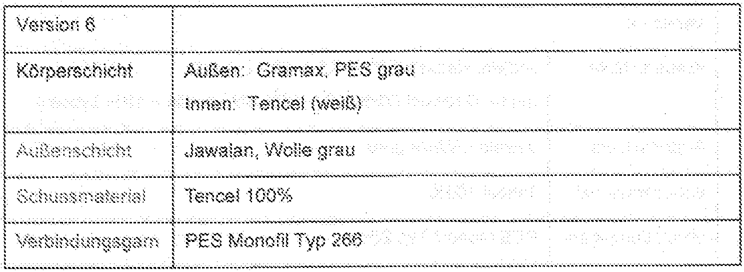

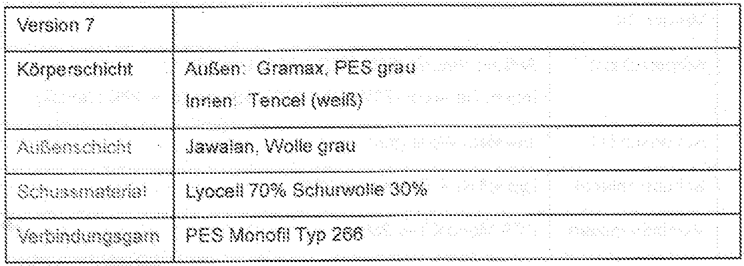

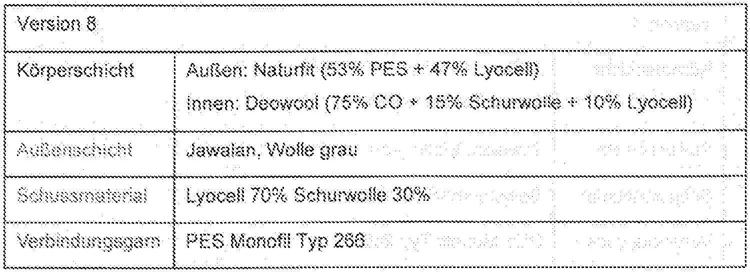

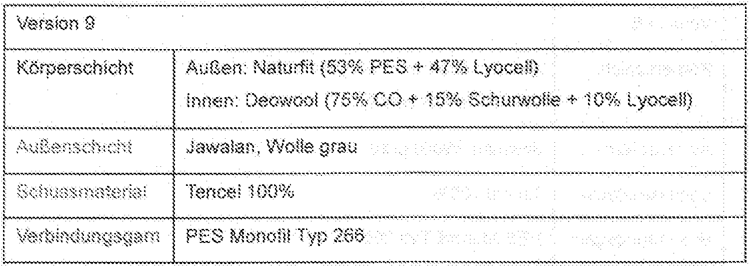

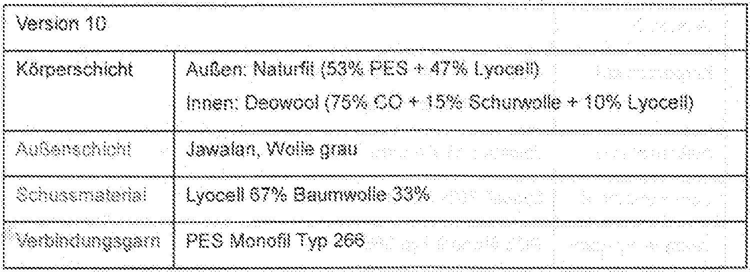

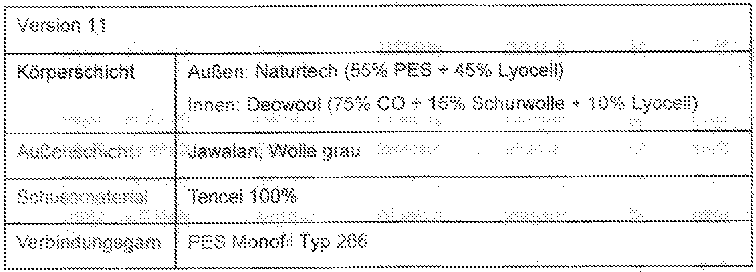

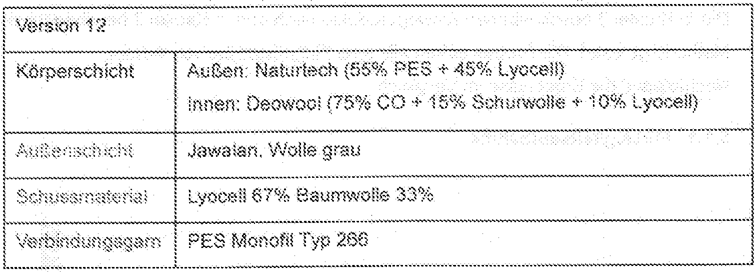

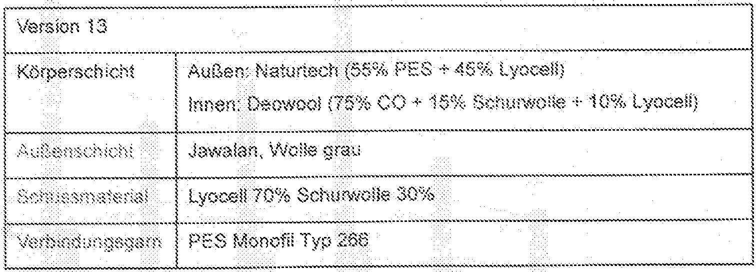

### 6.,3 Entwickelte Fläche

Nach einigen Vorversuchen hinsichtlich Haptik und Griff wurden am Ende 12 verschiedene Versionen getestet. Hier die Auswertung von 12 der 13 angedachten Materialtests. Die Version 5 wurde vor Testbegunn verworten.

### 6.3.1 Flüssigkeitsaufnahme

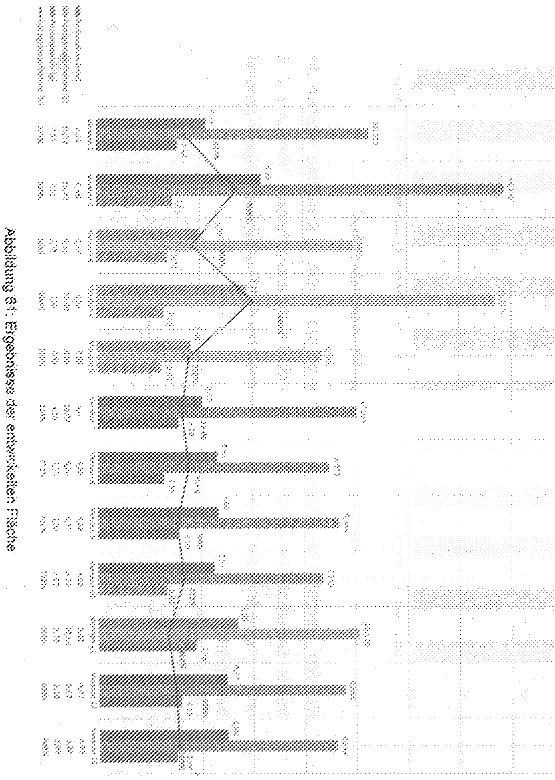

Die in Abbildung 81 zu sehenden Versionen 2 und 4 wurden jeweils mit dem selbst hergestellten Superabsorber-Gam produziert und weisen eine dementsprechende Materialstärke auf. Die Flüssigkeitsaufnahme ist durch den Einsatz der Superabsorberfasem deutlich erhöht.

Durch die Verwendung von dünneren Schussgamen in Version 6-13, konnte die Haptik der textilen Fläche deutlich verbessert werden. Der Flüssigkeitstransport ins Innere wurde durch den Einsatz von PES-Gam verbessert.

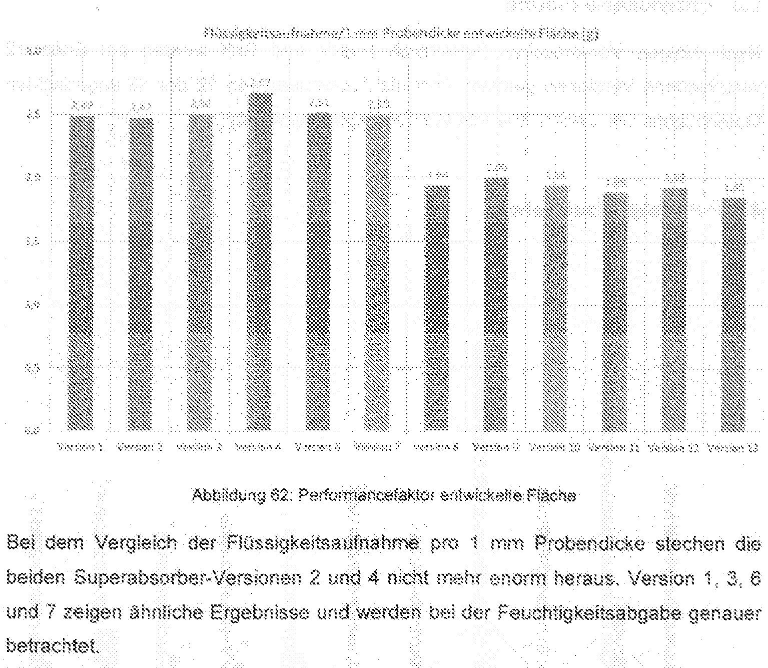

Vergleich Flüssigkeitsaufnahmeentwickelte Fläche und Mehrwegprodukte.

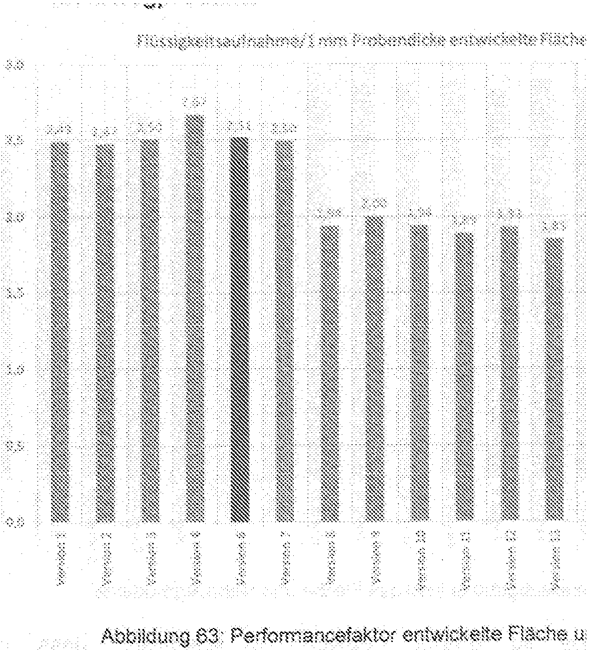

In Abbildung 63 wird der Vergleich des Performancewertes für die entwickelte Fläche und den Mehrwegprodukten miteinander verglichen. Die Flächen 1-4 werden dabei aufgrund Ihrer Steifigkeit/Haptik nicht weiter in Betracht gezogen. Die Herstellung eines neuartigen Superabsorber-Garns bedarf einer intensiveren Untersuchung in Bezug auf das im Garn verwendete Mischungsverhältnis der Superabsorber-Fasern, um möglichst optimale Materialeigenschaften zu erzielen. Die beiden besten Ergebnisse ohne Superabsorber-Garn erzielen die Versionen 6 und 7. Beide Versionen haben bei der Körperschicht außen ein Polyester und innen ein Lyocell-Garn. Lediglich beim Schussmaterial unterscheiden sich die beiden Versionen: Version 6 hat 100% Tencel im Schuss und Version 7 hat 70% Lyocell und 30% Schurwolle.

### Flüssigkeitsabgabe

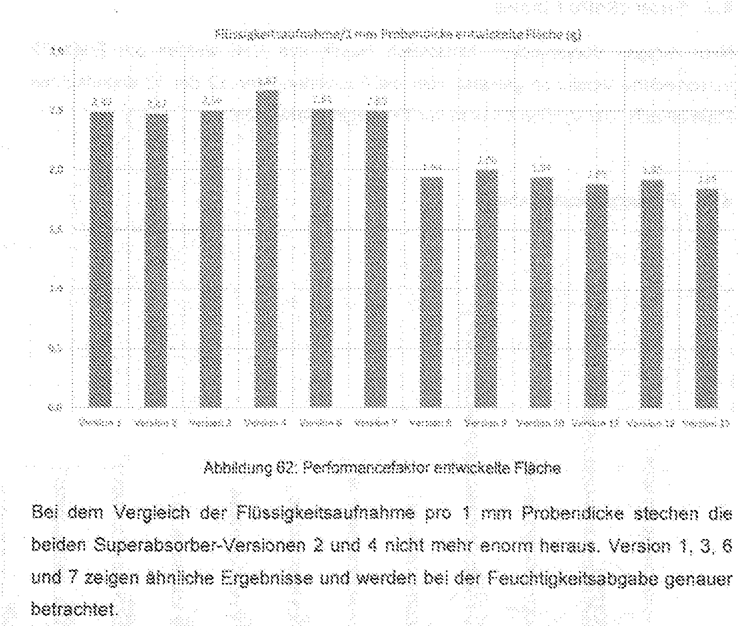

Bei der Flüssigkeitsabgabe ist erneut deutlich zu erkennen, dass die PES-Körperschicht bei den Versionen 6 und 7 eine Verringerung der Flüssigkeitsabgabe mit sich bringt. Das Vergleichsprodukt Protechdry gibt minimal mehr Flüssigkeit ab. Diese Abgabe bezieht sich auf die reale Flussigkeitsabgabe und nicht auf den Performancewert der Flüssigkeitsabgabe.

### Fazit

Es ist gelungen auf rein bindungstechnischer Basis ein Materialkonzept zu entwickeln, das für eine Vielfalt von Mehmveg-lnkontinenzartikeln und Produkten mit Flüssigkeitsmanagement weiter entwickelbar ist. Das aufgezeigte Materialkonzept ist wie ein Baukastensystem zu verstehen, das an unterschiedliche Anforderungsprofile angepasst werden kann. Die gewählte Variante in diesem vorliegenden Bericht kann in verschiedene Unterwäschetypen implementiert werden. Die Flächentechnologie Strick ermöglicht es zudem in formgerecht produzierten Flächen mit geschlossenen Kanten zu produzieren (Fully Fashioned oder Seamless). Zusätzliche Herstellungsschritte der folgenden Produkte (Zuschnitt, Nähen, Versäubern, Abdichten) können einspart werden, Folien und Beschichtungen werden unnötig. Der Abstandsbereich der entwickelten Fläche, bleibt nicht hohl, sondern wird mit einem voluminösen, Feuchtigkeit saugenden und bindenden Schussfaden gefüllt.

### Ausblick

Der Einsatz der textilen Fläche ist für Unterwäsche gedacht und muss für jede verschiedene Form der Unterwäsche erneut spezifiziert und angepasst werden. Dazu müssen Dinge wie Passform und Schnitt im Nachgang noch erarbeitet werden. Mehrweg-Inkontinenzeinlagen müssen natürlich mehrfach gewaschen werden. Daher sollten die empfohlenen Flächen diversen Waschtests unterzogen werden. Außerdem sollten die Flüssigkeitsaufnahmetests und -abgabetests nach standardisierten Waschzyklen wiederholt werden. Durch den Einsatz von natürlich funktionalen Faserstoffen wie Wolle und Lyocell hat das Produkt gute olfaktorische Eigenschaften. Daher sollte ein umfangreicher Geruchstest durchgeführt werden, um die Geruchsneutralisierung zu verifizieren und einzuordnen.

Die Forschungeergebnisse wurden ohne die Nennung fremder Marken übernommen.

Im folgenden werden, nicht einschränkend zu verstehende Ausführungsbeispiele der vorliegenden Erfindung anhand der Zeichnung besprochen. In dieser zeigen:
Fig. **1** schematisch, Grundaufbau der textilen Fläche für eine Verwendung am Körper (nicht erfindungsgemäß)
Fig. **2a** und Fig. **2b** schematisch, Grundaufbau der textilen Fläche für eine Verwendung am Körper mit fünf Materialschichten und einer Detailskizze der ersten Schicht (nicht erfindungsgemäß)
Fig. **3** schematisch, Grundaufbau der textilen Fläche für eine Verwendung am Körper mit sechs Materialschichten
Fig. **4** schematisch, Verfahren zur Herstellung einer Ausführungsform der textilen Fläche ohne Plattierung
Fig. 5 zeigt die Versetzung der Polfaden in den einzelnen Abfolgenschritten S3.

### Bezugszeichenliste:

1 textile Fläche
2 ableitende Körperschicht, erste Schicht
2a oberer Abschnitt der ersten Schicht, hydrophob
2b unterer Abschnitt der ersten Schicht, hydrophil
3 saugende und haltende Schicht, absorbierender Kern
3a hydrophobe Querfäden
4 blockierende Außenschicht, Außenseite der textilen Fläche
4a innerer Abschnitt der Aussenschicht
4b äusserer Abschnitt der Aussenschicht
5 hinteres Nadelbett
6 vorderes Nadelbett

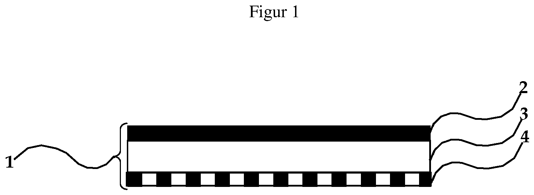

Fig.**1** zeigt schematisch, Grundaufbau der textilen Fläche für eine Verwendung am Körper.

Hier sind leicht die 3 funktionellen Schichten zu erkennen, aus welchen eine textile Fläche **1** aufgebaut sein kann. Die textile Fläche **1** zeigt in dieser Figur einen 3-Schichtaufbau, wobei jede der Schichten eine unterschiedliche Funktion ausübt. Für die Außenschicht **4,** also die Schicht, die vom Körper weg weist, wird in diesem Fall filzfähige Wolle gewählt. Das Filzen sorgt für eine Verdichtung des Materials. Zusammen mit den hydrophoben, wasserabweisenden Eigenschaften der Wolle liegt nun eine wasserdichte Fläche vor. Damit ist die Außenseite **4** flüssigkeitsdicht, es handelt sich also um eine blockierende Außenschicht **4.** Die vorliegende Flüssigkeit kann nicht nach aussen an die Bekleidung treten. Die ableitende Körperschicht **2,** welche direkt am Körper anliegt, soll die anfallende Flüssigkeit schnellstmöglich in das Innere der textilen Fläche **1** transportieren. Dort befindet sich die saugende und haltende Schicht **3,** welche die Flüssigkeit aufnimmt und hält. Die Funktionen dieser drei Schichten sind dem Grunde nach bei allen Ausführungsformen aufzufinden. So können weitere technische Merkmale zusätzliche Modifikationen und signifikante Verbesserungen einführen. Um die flüssigkeitstransportierende ableitende Körperschicht **2** möglichst dünn zu gestalten, wird die Plattiertechnik angewandt, anhand derer zwei Garne innerhalb der Schicht nochmals getrennt positioniert werden können: Das flüssigkeitstransportierende Garn wird dabei außen und das flüssigkeitshaltende Garn innen verwendet. Das flüssigkeitstransportierende Garn dient zudem dazu, ein möglichst trockenes Gefühl auf der Haut zu erzeugen und den Flüssigkeitsrücktransport zu verhindern.

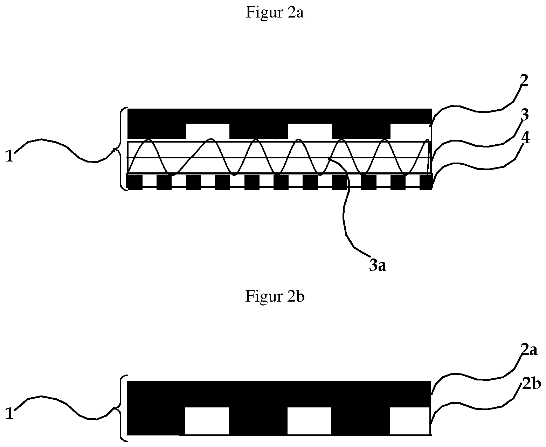

Fig. **2a** und Fig. **2b** zeigt schematisch den Grundaufbau der erfindungsgemäßen textilen Fläche für eine Verwendung am Körper mit fünf Materialschichten und einer Detailskizze der ersten Schicht. In dieser schematischen Zeichnung sind nun mehr Merkmale zu erkennen. Die Anzahl der oben genannten funktionellen Schichten ist im Prinzip gleich geblieben. Doch die ableitende Körperschicht **2,** welche direkt am Körper anliegt, ist in diesem Ausführungsbeispiel in zwei Abschnitte unterteilt. Sie besteht aus einem oberen hydrophoben Abschnitt **2a** und einem unteren hydrophilen Abschnitt **2b.** Die Aufteilung in die zwei Abschnitte wird durch den durchgezogenen **2a** bzw. grob gerasterten Strich **2b** angezeigt. In der Figur 2b wird nur die erste Schicht **2** vergrößert gezeigt. Der obere Abschnitt **2a** der ersten Schicht ist glatt gestrickt und mit dem unteren Abschnitt **2b** der ersten Schicht plattiert. Der untere Abschnitt **2b** ebenfalls glatt gestrickt ist. Der obere Abschnitt **2a** hat hydrophobe Eigenschaften, jegliche Art von hydrophoben Garn kann zu seinem Aufbau verwendet werden. Es wurden schon synthetische Materialien wie PET oder PA oder Merinowolle, Wollmischungen oder andere hydrophobierte Materialien und deren Kombinationen in beliebiger Zusammensetzung genannt. PET, also Polyethylenterephthalat ist als Textilfaser knitterfrei, reißfest und witterungsbeständig. Es nimmt aufgrund seiner hydrophoben Eigenschaften kaum Wasser auf. PA, ausgeschrieben Polyamid, ist allgemein auch als Nylon bekannt. Es zeichnet sich durch seine Zugfähigkeit und Festigkeit aus. Die ebenfalls hydrophob wirkende Merinowolle, ein Naturprodukt, kühlt und absorbiert den Geruch besonders gut. Der hydrophile Abschnitt **2b** der ersten Schicht besteht dementsprechend aus hydrophilem Garn, wie Tencel, Deocell, Deowool, Baumwolle, Modal, Bambus, Lyocell, Viskose und deren Kombinationen in beliebiger Zusammensetzung. Tencel oder Lyocell werden aus Cellulose gewonnen und weisen in faserförmiger Form eine hohe Trocken- und Nassfestigkeit auf. Sie sind weich und absorbieren die Feuchtigkeit sehr gut. Diese Eigenschaften prädestinieren diesen Stoff zur Auflage auf der Haut. Die erste Schicht **2** ist ja diejenige Schicht, die mit dem Körper Kontakt hat, also dem Körper zugewandt ist. Deocell ist als weiterverarbeitete Tencel-Faser zusätzlich auch noch antibakteriell. Modal ist ebenfalls ein Celluloseprodukt. Es ist aber im Vergleich mit Baumwolle oder anderen Stoffen glatter, saugfähiger, hitzebeständiger und hat eine höhere Festigkeit. Eine schnelle Absorption von Flüssigkeit auf der dem Körper zugewandten Seite ist aufgrund der porösen Natur des Gestrickes und des hohen Absorptionsgradienten möglich, der durch Kombinieren von Materialien mit entgegengesetzten Fasereigenschaften in einer einzigen Schicht gebildet wird. Der Absorptionsprozess verläuft wie folgt: Der obere Abschnitt der ersten Schicht **2a** ist hydrophob ausgestaltet Er liegt direkt zum Körper hin, während der untere Abschnitt **2b** hydrophil ist. Durch diesen Aufbau wird der hohe Absorptionsgradient erzeugt und die ankommende Flüssigkeit wird schnell in Richtung zweite Schicht **3** transportiert. Die erste Schicht **2** speichert somit keine signifikante Flüssigkeitsmenge und fühlt sich trocken an. Ein Rückfluss zum Körper hin, gegen den Absorptionsgradienten, ist nicht möglich. Die absorbierte Flüssigkeit liegt nicht mehr in der Nähe des Körpers vor. Dies sorgt für den hohen Tragekomfort der vorliegenden Erfindung. Sie wird zu großen Anteilen auch mit Hilfe der Schwerkraft in die zweite Schicht 3 hineintransportiert.

Die zweite Schicht 3, auch als Zwischenschicht bezeichnet, da sie zwischen der ersten und dritten Schicht liegt, weist hydrophobe Querfäden 3a auf. Entlang dieser hydrophoben Querfäden 3a wird die absorbierte Flüssigkeit weitertransportiert, hin zum absorbierenden Kern 3. Dieser ist stark hydrophil. Er besteht aus einem hydrophilen Füllgarn und dient damit als hydrophiles Inlay, welches die Flüssigkeit aufnimmt und zurückhält. Das Füllgarn wird lose gesponnen und/oder mit zusätzlichen Mikrofasern versehen. Dies sorgt für eine erweiterte Oberfläche mit dementsprechend hohem Absorptionsvermögen. Entlang der hydrophoben Querfäden 3a kann die Flüssigkeit auch nicht wieder zurück. Die hydrophoben Querfäden 3a verbinden in nicht erfindungsgemäßen Ausgestaltungen die erste Schicht 2 mit der dritten Schicht 4, so dass diese aneinanderhängen. Sie sind in dieser Figur als gewellte Linie dargestellt Das hydrophile Füllgam besteht aus Tencel, Deocel, Deowool, Baumwolle, Modal, Bambus, Lyocell, Viskose, Natriumpolyarcylat, Kohlenstoffkristallen, Monofilamenten, Multifilament; losen Fasern, Mikrofasem, Garnen aus Superabsorberfasem und deren Kombinationen in beliebiger Zusammensetzung. Die hydrophoben Querfäden bestehen aus synthetischen Monofilamenten oder Multifilamenten (PET, PA, PP) oder aber auch Naturstoffe wie Leinen oder Hanf.

Zwischen der ersten Schicht 2 und der zweiten Schicht 3 liegt in nicht erfindungsgemäßen Ausgestaltungen ein schmaler Spalt vor. Dieser wird durch die vertikal verlaufenden Querfäden 3a erzeugt. Durch diesen Raum wird die Atmungsaktivität des Gestrickes im Ganzen erhöht Zusätzlich wird eine schnellere Absorption mit nachfolgende Trocknung der aufgesaugten Flüssigkeit in der ersten Schicht 2 unterstützt Die Flüssigkeit gelangt so noch schneller in den absorbierenden Kem 3 und verbleibt dort Die erste und zweite Schicht 3 sind somit durch den schmalen Spalt auch räumlich getrennt.

Die dritte Schicht 4, die blockierende Außenschicht, verhindert in nicht erfindungsgemäßen Ausgestaltungen das Austreten der absorbierten

Flüssigkeit aus dem Gestrick. Diese Schicht ist hydrophob. Sie kommt mit dem Körper nicht in Berührung. In dieser vorliegenden Ausführungsform besteht die Schicht nur aus einem Abschnitt, nämlich Wolle, die nach dem Strickprozess gefilzt wird. Es können aber auch noch weitere wasserdichte Materialien zugesetzt werden, um die wasserdichten Eigenschaften zu verstärken. Wichtig ist nur die Erstellung einer hydrophoben Barriere.

In dieser Ausführungsform liegt ein waschbares, wiederverwendbares, facettenreiches, gestricktes Textil vor, das gewährleistet, dass unerwünschte Flüssigkeiten, Feuchtigkeit und Gerüche vom Körper weg adsorbiert werden, damit wird die Gesundheit und das Hygienebedürfnis des Trägers gefördert.

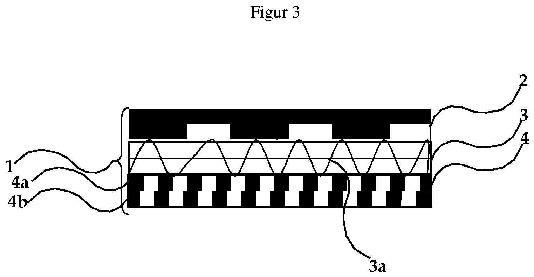

Fig. **3** zeigt schematisch den Grundaufbau der textilen Fläche für eine Verwendung am Körper mit sechs Materialschichten. Im oberen Teil gleichen sich die Figuren 2 und 3, aber die dritte Schicht **4** kann auch noch aus 2 Gestrickschichten (Abschnitten) **4a, 4b** aufgebaut sein. Dies dient zur Erhöhung der Feuchtigkeitsbeständigkeit bei der Verwaltung von größeren Flüssigkeitsmengen. Zusätzlich zu dem Abschnitt (Schicht) **4a,** der aus hydrophobem Material wie Wolle besteht, wird nun eine weitere Schicht (Abschnitt) **4b** hinzugefügt. Diese weitere Schicht besteht in diesem Ausführungsbeispiel aus einem niedrigschmelzenden thermoplastischen Polymer. Dieses thermoplastische Polymer wird während des Strickvorganges als Faden miteingestrickt und anschließend geschmolzen. Hierdurch werden zusätzlich nochmals die Poren des Gestrickes verschlossen und versiegelt. Diese Maßnahme wirkt also zusätzlich zur gefilzten Wolle, welche ja den inneren Abschnitt **4a** der dritten Schicht (in dieser Ausführungsform der Erfindung) bildet. Diese Ausführungsform der erfinderischen textilen Fläche zeigt nochmals deutlich, dass hier ein waschbares mehrschichtiges, gestricktes Textil vorliegt. Dies weist hochabsorbierende Eigenschaften auf, die auf der Kombination von Materialien mit entgegengesetzten Eigenschaften beruhen, die als Zusammensetzung poröser Schichten aufgebaut sind, die wässrige Flüssigkeiten vom Körper weg und in Richtung des saugfähigen Kerns des Gestrickes transferieren. Die Schichten und Abschnitte mit ihrem jeweiligen Material und die Physik des Flüssigkeitstransportes ist in der Beschreibung und den Beispielen dargelegt worden. Es sei hier nochmals darauf hingewiesen, dass die Produktzusammensetzungen den jeweiligen Anforderungen angepasst werden können. Es können Textilveredelungsverfahren zu einer weiteren Verbesserung der Fläche vorgenommen werden. Die Hygienestandards können durch Einfügen weiterer antibakterieller und/oder geruchsneutraler bzw. geruchsschluckender Fasern erhöht werden. Dies kann für jede Schicht und jeden Abschnitt einzeln oder zusammen vorgenommen werden. So stellt das Einspinnen von Metallionenfäden zum Erhöhen des antibakteriellen Charakters der textilen Fläche kein Problem dar. Hochwertige Kohlenstoffkristalle und Kohlenstoffnetze sowie Naturstoffe wie die geruchsneutralen Yamwurzeln können Bestandteil der textilen Fläche sein und in jeder Schicht (Abschnitt) des gestrickten Konstruktes verwendet werden.

Die textile Fläche kann als ein durchgehendes Stück auf einer Flachbett- oder Rundstrickmaschine gestrickt werden. Die textile Fläche kann hierdurch bereits in jeder gewünschten Form gestrickt werden, somit können mögliche Konfektionsschritte übersprungen oder gar ganz weggelassen werden. Die hier verwendete Stricktechnik erlaubt es sogar vollständige konfektionierte und ausgeformte Stoffteile in das Endprodukt einzufügen. Die Methode ist umweltfreundlich, wirtschaftlich und nachhaltig. Es fallen nämlich keine Reste an, da hier nicht zugeschnitten werden muss. Es sind auch nach dem Strickvorgang keine weiteren Arbeitsschritte mehr notwendig. Das Produkt liegt vollkommen gefertigt vor. So werden unter anderem keine Stoffkanten mehr zu schließen sein.

Folgende Tabelle gibt die einzelnen Schichten und Abschnitte, sowie deren eine Auswahl des zu verwendeten Materials an:

| Abfolge | Abfolge, detailiert | Material | Eigenschaft |
|---|---|---|---|
| flüssigkeitsableitende Körperschicht **2,** | Plattierfaden **2a,** oberer Abschnitt | PET oder PA oder Merinowolle, Wollmischungen oder andere hydrophobierte Materialien und deren Kombinationen in beliebiger Zusammensetzung | hydrophob |
| erste Schicht | | | |
| flüssigkeitsableitende Körperschicht **2** | Grundfaden **2b,** | hydrophiles Garn, wie Tencel, Deocell, Deowool, Baumwolle, Modal, Bambus, Lyocell, Viskose und deren Kombinationen in beliebiger Zusammensetzung. | hydrophil |
| | unterer Abschnitt | | |
| erste Schicht | | | |
| Zwischenschicht **3,** zweite Schicht, Mittelschicht | Füllfaden **3a,** | synthetische Monofilamente oder Multifilamente (PET, PA, PP) oder aber auch Naturstoffe wie Leinen oder Hanf | hydrophob |
| | hydrophober Querfaden | | |
| Zwischenschicht **3,** zweite Schicht, Mittelschicht | Hydrophiles Füllgarn, | Tencel, Deocel, Deowool, Baumwolle, Modal, Bambus, Lyocell, Viskose, Natriumpolyarcylat, Kohlenstoffkristalle, Monofilamente, Multifilament, lose Fasern, Mikrofasern, Garne aus Superabsorberfasern und deren Kombinationen in beliebiger Zusammensetzung | hydrophil |
| | Hydrophiles Inlay | | |
| Dritte Schicht, blockierende Außenschicht **4** | Grundfaden **4a,** | Wolle, Wollmischungen, Baumwolle, Polyamid, Polyester, Modal, Tencel, optional Schmelzgarn (niedrigschmelzendes thermoplastisches Garn) und deren Kombinationen in beliebiger Zusammensetzung | hydrophob |
| Dritte Schicht, blockierende Außenschicht **4** | Plattierfaden **4b** | Wolle, Wollmischungen optional beides gefilzt oder weiterhin optional Schmelzgarn, Polyamid und deren Kombinationen in beliebiger Zusammensetzung | hydrophob |

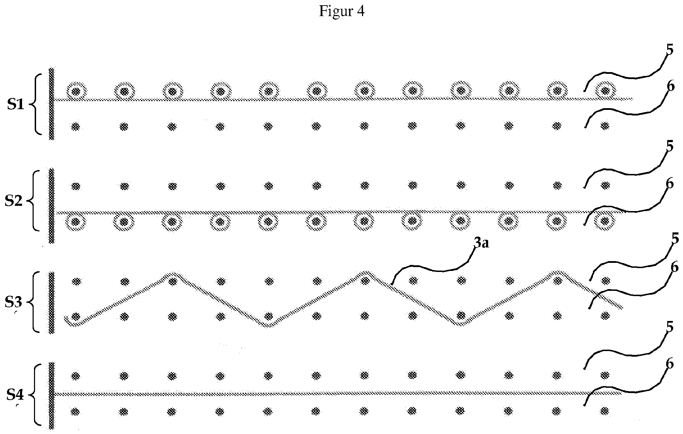

Fig. 4 zeigt schematisch das Verfahren zur Herstellung einer Ausführungsform der textilen Fläche ohne Plattierung. Die textile Fläche kann auf einer Flach- oder Rundstrickmaschine hergestellt werden. In diesem Ausführungsbeispiel wird die erfindungsgemäße textile Fläche von einer Rundstrickmaschine gefertigt. Die dreidimensionale mehrschichtige Strickstruktur wird auf der Rundstrickmaschine durch eine bestimmte Abfolge von Stichen/Schlaufen (Maschen) erzeugt. Hierzu kann man eine automatische Strickmaschine verwenden, welche programmiert werden muss.

Die Figur ist so aufgebaut, dass die zeitliche Abfolge von oben nach unten dargestellt ist. Man sieht deutlich das hintere Nadelbett **5** und das vordere Nadelbett **6.** Die Nadeln sind als Punkte dargestellt. In einem ersten Abfolge-Schritt **S1** wird ein hydrophiles Garn im Zylinder gestrickt. Der Fadenführer läuft beispielsweise von links nach rechts. Hiernach wird in einem zweiten Abfolge-Schritt **S2** ein hydrophobes Garn im Zifferblatt gestrickt. In dem folgenden dritten Abfolge-Schritt **S3** werden die Querfäden **3a** eingestrickt. Das hydrophobe Monofilament wird im Zylinder und Zifferblatt eingestrickt, wobei nur jede 4.te Nadel so bewegt wird. Und so einen Henkel erzeugt. Anschließend wird das hydrophile Füllmaterial zwischen die Schichten gelegt. Werden die erste und die dritte Schicht so gestrickt, dass die Schichten jeweils aus den 2 Abschnitten bestehen, dann wird in einem ersten Abfolge-Schritt **S1** ein hydrophiles und eine hydrophobes Garn im Zylinder gestrickt, wobei das hydrophobe Garn nach aussen weist. Die beiden Garne werden gleichzeitig unter Verwendung eines Plattierungsträgers **(also eines Fadenführers)** gestrickt, um auf jeder Nadel einen plattierten glatten Stich zu bilden, bei dem die Nadeln eine vollständige Bewegung ausführen, um vollständige Maschen zu erzeugen. Hiernach wird in einem zweiten Abfolge-Schritt **S2** ein hydrophobes Garn und ein niedrigschmelzender Thermoplast (Schmelzgarn) im Zifferblatt gestrickt. Der niedrigschmelzende Thermoplast vom Zifferblatt aus gesehen nach aussen weist. In dem folgenden dritten Abfolge-Schritt **S3** werden die Querfäden eingestrickt. Das hydrophobe Monofilament wird im Zylinder und Zifferblatt eingestrickt, wobei nur jede 4.te Nadel so bewegt wird, dass sie nicht die Oberflächen der textilen Fläche durchstößt, sondern nur einen Henkel erzeugt. Anschließend wird im Abfolgeschritt **S4** das hydrophile Füllmaterial zwischen die Schichten gelegt. Es kann nach dem Strickvorgang nicht mehr aus der textilen Fläche genommen werden oder herausfallen. Die Abfolgeschritte **S1** und **S2** können auch in umgekehrter Reihenfolgen vorgenommen werden, danach ist der Abfolgeschritt **S3** und folgend **S4** zu tätigen.

Die im 3.ten Abfolgeschritt eingebrachten Querfäden (Polfäden) können auch in dem darauf darauffolgenden Abfolgschritt S3 gegenüber dem schon eingebrachten Polfäden versetzt sein.

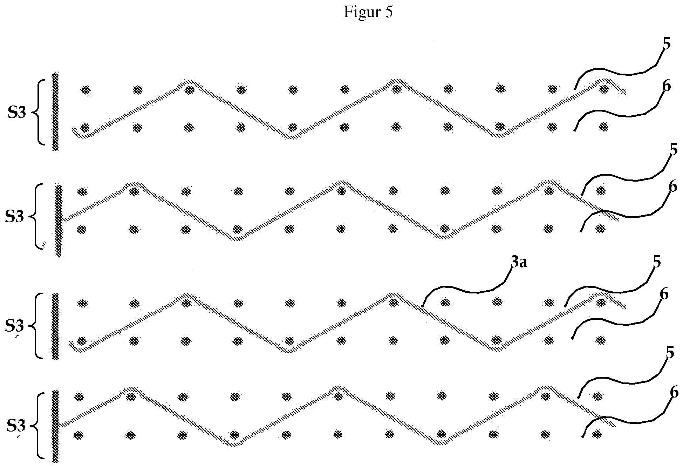

Figur 5 zeigt die Versetzung der Polfäden in den einzelnen Abfolgenschritten S3. In diesem Fall sieht man die Verschiebung (Versatz) um eine Nadel im Nadelbett 5. Es können aber auch andere Verschiebungen möglich sein. Ein solcher Versatz sorgt für eine glattere Fläche. Dies macht sich besonders bei festeren Stoffen (textilen Flächen) bemerkbar.

## Patentansprüche

1. Textile Fläche (1), welche als dreidimensionales mehrschichtiges Strickkonstrukt vorliegt, welches mindestens drei Schichten aufweist, **dadurch gekennzeichnet, dass** die erste und die dritte Schicht jeweils mindestens zwei Abschnitte aufweist, wobei
• die erste Schicht (2) die Flüssigkeit aufnimmt und zur Zwischenschicht (3) transportiert und wobei die erste Schicht (2) einen oberen Abschnitt (2a), der hydrophob ist, und einen unteren Abschnitt (2b), der hydrophil ist, aufweist und beide Abschnitte glatt gestrickt sind und die Abschnitte plattiert,
• die zweite Schicht (3), nämlich die Zwischenschicht, die Flüssigkeit aufnimmt und zurückhält, wobei die Zwischenschicht hydrophobe Querfäden (3:1), welche die erste und die dritte Schicht miteinander verbinden und ein hydrophiles Inlay aufweist und
• die dritte Schicht (4), die verhindert, dass die Flüssigkeit aus dem Stoff entweicht und wobei die dritte Schicht (4) einen oberen Abschnitt, der hydrophob ist und einen unteren Abschnitt, der wasserdicht ist, aufweist und beide Abschnitte glatt gestrickt sind und die Abschnitte plattiert werden.

2. Textile Fläche (1) nach Anspruch 1, **dadurch gekennzeichnet, dass**
• der obere Abschnitt (2a) der ersten Schicht aus hydrophoben Garn besteht, welche aus der folgenden Aufzählung ausgewählt ist, nämlich synthetische Materialien wie PET oder PA oder Merinowolle, Wollmischungen oder andere hydrophobierte Materialien und deren Kombinationen in beliebiger Zusammensetzung,
• der untere Abschnitt (2b) der ersten Schicht aus hydrophilem Garn besteht, welche aus der folgenden Aufzählung ausgewählt ist, nämlich Tencel, Deocell, Deowool, Baumwolle, Modal, Bambus, Lyocell, Viskose und deren Kombinationen in beliebiger Zusammensetzung,
• die hydrophoben Querfäden (321) aus jeder Art von hydrophobem Garn besteht, insbesondere Polyester, synthetischen Monofilamenten oder Multifilamenten, aber auch aus Naturstoffen wie Leinen oder Hanf,
• das hydrophile Inlay aus jeder Art von hydrophilem Garn oder Faser besteht, insbesondere Tencel, Deocell, Deowool, Baumwolle, Modal, Bambus, Lyocell, Viskose, Natriumpolyacrylat, Kohlenstoffkristalle, Monofilamente, Multifilament, lose Fasern, Mikrofasern, Garne aus Superabsorberfasern und deren Kombinationen in beliebiger Zusammensetzung,
• der obere Abschnitt der dritten Schicht aus hydrophobem Garn besteht, welcher aus der folgenden Aufzählung ausgewählt ist, nämlich Wolle, Wollmischungen, Baumwolle, Polyamid, Polyester, Modal, Tencel, optional Schmelzgarn und deren Kombinationen in beliebiger Zusammensetzung
• der untere Abschnitt der dritten Schicht aus hydrophobem Garn besteht, welcher aus der folgenden Aufzählung ausgewählt ist, nämlich Wolle, Wollmischungen optional beides gefilzt oder weiterhin optional Schmelzgarn, Polyamid und deren Kombinationen in beliebiger Zusammensetzung.

3. Verwendung der textilen Fläche (1) gemäß der Ansprüche 1 bis 2 im medizinischen Bereich, insbesondere als OP-Unterlagen, Bettunterlagen und Verbandsmittel, Verbandsmaterial.

4. Verwendung der textilen Fläche (1) gemäß der Ansprüche 1 bis 2 im Hygienebereich, insbesondere als Inkontinenz- bzw. Menstruationsbekleidungen, insbesondere Inkontinenzhosen und Menstruationshosen, Unterwäsche, schweißaufsaugende Textilien, Regenbekleidung.

5. Verwendung der textilen Fläche (1) gemäß der Ansprüche 1 bis 2 im veterinärmedizinischen und Tier-Hygiene-Bereich, insbesondere Tierdecken, OP-Unterlagen, Verbandsmaterial, Menstruationshosen.

6. Verwendung der textilen Fläche (1) gemäß der Ansprüche 1 bis 2 im täglichen Hygienebereich, insbesondere Gardinen, Tischdecken, Bezüge von Sitzmöbeln aller Art, Überwurfdecken.

7. Verfahren zur Herstellung einer wiederverwendbaren dreidimensionalen textilen Fläche (1), **dadurch gekennzeichnet, dass**
• in einem ersten Schritt S1 eine erste Schicht aus einem hydrophoben und einem hydrophilen Garn gestrickt wird, wobei das hydrophobe Garn nach außen gerichtet ist und das hydrophile Garn nach innen weist und
• in einem zweiten Schritt S2 eine zweite Schicht aus einem hydrophoben Garn und einem hydrophoben Garn zusammen mit einem niedrigschmelzenden thermoplastischen Faden gestrickt wird, wobei das hydrophobe Garn nach Innen weist und im Falle des Vorhandenseins der niedrigschmelzenden thermoplastischen Faden nach aussen weist,
• folgend S3 wird/werden ein/mehrere Querfaden/Querfäden gestrickt, wobei sich nur jede 3.te oder 4.te Nadel auf halber Strecke bewegt, wodurch ein Henkel erzeugt wird, welcher die erste und zweite Schicht zusammenhält
• danach S4 wird ein hydrophiles Füllgarn zwischen den beiden Schichten platziert
• ein oberer Abschnitt und ein unterer Abschnitt der ersten Schicht sowie ein oberer und ein unterer Abschnitt der dritten Schicht werden plattiert.

8. Verfahren zur Herstellung einer wiederverwendbaren dreidimensionalen textilen Fläche (1) nach dem Anspruch 8, **dadurch gekennzeichnet, dass** nach den ersten beiden Schritten die Querfäden mit Versatz gestrickt werden.

9. Verfahren zur Herstellung einer wiederverwendbaren dreidimensionalen textilen Fläche (1), **dadurch gekennzeichnet**, die Abfolgeschritte S1, S2, S3, S4 in allen nur denkbaren Abfolgen hintereinander abfolgen.

## Claims

1. A textile sheet (1) which is in the form of a three-dimensional multi-layer knit structure having at least three layers, **characterised in that** the first and third layers each have at least two sections, wherein
• the first layer (2) receives the liquid and transports it to the intermediate layer (3) and wherein the first layer (2) has an upper portion (2a) which is hydrophobic and a lower portion (2b) which is hydrophilic and both portions are smooth knitted and the portions are plated,
• the second layer (3), namely the intermediate layer, absorbs and retains the liquid, wherein the intermediate layer has hydrophobic transverse threads (3:1) connecting the first and third layers and is a hydrophilic inlay, and
• the third layer (4) prevents the liquid from escaping from the fabric and wherein the third layer (4) has an upper portion that is hydrophobic and a lower portion that is waterproof and both portions are smooth knitted and the portions are plated.

2. The textile sheet (1) according to claim 1, **characterised in that**
• the upper portion (2a) of the first layer is made of hydrophobic thread selected from the following list, namely synthetic materials such as PET or PA or merino wool, wool blends or other hydrophobic materials and combinations thereof in any composition,
• the lower portion (2b) of the first layer is made of hydrophilic thread selected from the following list, namely Tencel, Deocell, Deowool, cotton, Modal, bamboo, Lyocell, viscose and combinations thereof in any composition,
• the hydrophobic transverse threads (321) are made of any type of hydrophobic thread, in particular polyester, synthetic monofilaments or multifilaments, but also of natural materials such as linen or hemp,
• the hydrophilic inlay consists of any type of hydrophilic thread or fibre, in particular Tencel, Deocell, Deowool, cotton, Modal, bamboo, Lyocell, viscose, sodium polyacrylate, carbon crystals, monofilaments, multifilament, loose fibres, microfibres, threads made of superabsorbent fibres and combinations thereof in any composition,
• the upper portion of the third layer is made of hydrophobic thread selected from the following list, namely wool, wool blends, cotton, polyamide, polyester, Modal, Tencel, optionally meltblown thread and combinations thereof in any composition
• the lower portion of the third layer consists of hydrophobic thread selected from the following list, namely wool, wool blends optionally both felted or further optionally melt thread, polyamide and combinations thereof in any composition.

3. Use of the textile sheet (1) according to claims 1 to 2 in the medical field, in particular as surgical underlays, bed underlays and bandages, dressing material.

4. Use of the textile sheet (1) according to claims 1 to 2 in the hygiene sector, in particular as incontinence or menstrual clothing, in particular incontinence pants and menstrual pants, underwear, sweat-absorbent textiles, rainwear.

5. Use of the textile sheet (1) according to claims 1 to 2 in the veterinary and animal hygiene sector, in particular animal blankets, surgical underlays, dressing material, menstrual pants.

6. Use of the textile sheet (1) according to claims 1 to 2 in the daily hygiene sector, in particular curtains, tablecloths, covers for seating furniture of all kinds, throw blankets.

7. A Method for producing a reusable three-dimensional textile sheet (1), **characterised in that**
• in a first step S1, a first layer is knitted from a hydrophobic and a hydrophilic thread, the hydrophobic thread facing outwards and the hydrophilic thread facing inwards, and
• in a second step S2, a second layer is knitted from a hydrophobic thread and a hydrophobic thread together with a low-melting thermoplastic thread, the hydrophobic thread facing inwards and, in the case of the presence of the low-melting thermoplastic thread, facing outwards,
• following S3, the transverse thread(s) is/are knitted, wherein only every 3rd or 4th needle moves halfway, creating a handle that holds the first and second layer together
• then S4, a hydrophilic filling yarn is placed between the two layers
• an upper section and a lower section of the first layer and an upper and a lower section of the third layer are plated.

8. The method for producing a reusable three-dimensional textile sheet (1) according to claim 8, **characterised in that** after the first two steps, the transverse threads are knitted with offset.

9. Method for producing a reusable three-dimensional textile sheet (1), **characterised in that** the sequence steps S1, S2, S3, S4 follow one another in all conceivable sequences.

## Revendications

1. Surface textile (1) qui se présente sous la forme d'une construction tricotée multicouche tridimensionnelle, qui comprend au moins trois couches, **caractérisée en ce que** la première et la troisième couche comprennent chacune au moins deux sections, où
• la première couche (2) reçoit le liquide et le transporte vers la couche intermédiaire (3) et dans lequel la première couche (2) comprend une partie supérieure (2a) qui est hydrophobe et une partie inférieure (2b) qui est hydrophile, et les deux parties sont tricotées de manière lisse et les parties sont plaquées,
• la deuxième couche (3), à savoir la couche intermédiaire, absorbe et retient le liquide, la couche intermédiaire comprenant des filaments transversaux hydrophobes (3:1) reliant la première et la troisième couche et un inlay hydrophile, et
• la troisième couche (4) qui empêche le liquide de s'échapper du tissu et dans lequel la troisième couche (4) comprend une partie supérieure qui est hydrophobe et une partie inférieure qui est imperméable à l'eau, et les deux parties sont tricotées de manière lisse et les parties sont plaquées.

2. La surface textile (1) selon la revendication 1, **caractérisée en ce que**
• la partie supérieure (2a) de la première couche est constituée de fil hydrophobe choisi parmi la liste suivante, à savoir des matériaux synthétiques tels que le PET ou le PA ou la laine mérinos, des mélanges de laine ou d'autres matériaux hydrophobes et leurs combinaisons dans n'importe quelle composition,
• la partie inférieure (2b) de la première couche est constituée de fil hydrophile choisi parmi l'énumération suivante, à savoir Tencel, Deocell, Deowool, coton, modal, bambou, lyocell, viscose et leurs combinaisons dans n'importe quelle composition,
• les fils transversaux hydrophobes (321) sont constitués de tout type de fil hydrophobe, notamment de polyester, de monofilaments ou de multifilaments synthétiques, mais aussi de matières naturelles telles que le lin ou le chanvre,
• l'inlay hydrophile est constitué de tout type de fil ou de fibre hydrophile, notamment Tencel, Deocell, Deowool, coton, modal, bambou, lyocell, viscose, polyacrylate de sodium, cristaux de carbone, monofilaments, multifilaments, fibres en vrac, microfibres, fils de fibres superabsorbantes et leurs combinaisons dans n'importe quelle composition,
• la partie supérieure de la troisième couche est constituée de fil hydrophobe choisi parmi la liste suivante, à savoir laine, mélanges de laine, coton, polyamide, polyester, modal, tencel, éventuellement fil fondu et leurs combinaisons dans n'importe quelle composition
• la partie inférieure de la troisième couche est constituée de fil hydrophobe choisi parmi la liste suivante, à savoir laine, mélanges de laine éventuellement feutrés tous les deux ou en outre éventuellement fil fondu, polyamide et leurs combinaisons dans n'importe quelle composition.

3. Utilisation de la surface textile (1) selon les revendications 1 à 2 dans le domaine médical, notamment comme alèse de bloc opératoire, alèse de lit et pansement, matériel de pansement.

4. Utilisation de la surface textile (1) selon les revendications 1 à 2 dans le domaine de l'hygiène, notamment comme vêtements d'incontinence ou de menstruation, en particulier des culottes d'incontinence et des culottes de menstruation, des sous-vêtements, des textiles absorbant la transpiration, des vêtements de pluie.

5. Utilisation de la surface textile (1) selon les revendications 1 à 2 dans le domaine de la médecine vétérinaire et de l'hygiène animale, en particulier des couvertures pour animaux, des dessous de salle d'opération, des pansements, des culottes menstruelles.

6. Utilisation de la surface textile (1) selon les revendications 1 à 2 dans le domaine de l'hygiène quotidienne, en particulier les rideaux, les nappes, les housses de sièges de tous types, les couvre-lits.

7. Le procédé de fabrication d'une surface textile tridimensionnelle réutilisable (1), **caractérisé en ce que**
• dans une première étape S1, une première couche est tricotée à partir d'un fil hydrophobe et d'un fil hydrophile, le fil hydrophobe étant dirigé vers l'extérieur et le fil hydrophile étant dirigé vers l'intérieur, et
• dans une deuxième étape S2, une deuxième couche est tricotée à partir d'un fil hydrophobe et d'un fil hydrophobe conjointement avec un fil thermoplastique à bas point de fusion, le fil hydrophobe étant tourné vers l'intérieur et, en cas de présence du fil thermoplastique à bas point de fusion, vers l'extérieur,
• ensuite S3, un/des fil(s) transversal(aux) est/sont tricoté(s), avec seulement chaque 3ème ou 4ème aiguille se déplaçant à mi-chemin, créant ainsi une anse qui maintient la première et la deuxième couche ensemble
• ensuite S4, un fil de remplissage hydrophile est placé entre les deux couches
• une partie supérieure et une partie inférieure de la première couche ainsi qu'une partie supérieure et une partie inférieure de la troisième couche sont plaquées.

8. Le procédé de fabrication d'une surface textile tridimensionnelle réutilisable (1) selon la revendication 8, **caractérisé en ce que**, après les deux premières étapes, les fils transversaux sont tricotés avec un décalage.

9. Procédé de fabrication d'une surface textile tridimensionnelle réutilisable (1), **caractérisé en ce que** les étapes de séquence S1, S2, S3, S4 se succèdent dans toutes les séquences imaginables.
